# EUROPEAN PATENT SPECIFICATION

(11) **EP 4 017 519 B1**
(45) Date of publication and mention of the grant of the patent: **05.03.2025**
(21) Application number: 20764591.2
(22) Date of filing: 21.08.2020
(51) Int. Cl.: A61K 38/17, A61K 38/39, A61P 17/02

(54) **TROPOELASTIN FOR USE IN TREATMENT OF ACNE SCARRING**
TROPOELASTIN ZUR VERWENDUNG BEI DER BEHANDLUNG VON AKNENARBEN
TROPOÉLASTINE DESTINÉE À ÊTRE UTILISÉE DANS LE TRAITEMENT DE LA CICATRISATION DE L'ACNÉ

(30) Priority: 23.08.2019 US 201962891232 P
(43) Date of publication of application: 29.06.2022
(73) Proprietor: Allergan Pharmaceuticals International Limited, Dublin, D17 E400 (IE)
(72) Inventor: DANIELS, Robert, Sydney, New South Wales NSW 2024 (AU); COLLINS, Caroline, Dublin, D 17 E400 (IE); ROBERTS, John St. Clair, Suffolk IP29 5RD (GB); WESTWATER, John, Dublin, D 17 E400 (IE)
(74) Representative: Cohausz & Florack
(86) International application number: PCT/EP2020/073516
(87) International publication number: WO 2021/037733

(56) References cited:
- WO-A1-2010/102337
- WO-A1-2013/044314
- WO-A1-2015/150332
- WO-A1-94/14958

## Description

### FIELD

This disclosure relates to methods of reducing the appearance of acne scars, such as depressions, ice pick scars, rolling atrophic acne scars and boxcar scars using a composition that comprises tropoelastin.

### BACKGROUND

Reference to any prior art in the specification is not an acknowledgment or suggestion that this prior art forms part of the common general knowledge in any jurisdiction or that this prior art could reasonably be expected to be understood, regarded as relevant, and/or combined with other pieces of prior art by a skilled person in the art.

Acne scars may be a result of infected blemishes caused by clogged skin pores that are engorged with excess oil, dead skin cells and bacteria. The pore swells, causing a break in the follicle wall. Shallow lesions are usually minor and may heal quickly. But if there is a deep break in the wall of the pore, infected material can spill out into surrounding tissue, creating a deeper lesion. Thus, the skin may attempt to repair these lesions by forming new collagen fibers. In some cases, a visible scar can be in a prominent location, such as the face, neck and chest which may be an annoyance to a person. There are multiple variables that may affect the severity of the scarring, such as the thickness and coloring of the scars. These repairs may not be as smooth and flawless as the original skin. Additionally, healing may lead to unwanted coloring of the skin within the scar.

There are several types of acne scars. Without being limiting, there are hypertrophic, ice pick scarring, boxcar type scarring, rolling atrophic scarring and depressed scarring. In some cases, the scarring can lead to discoloration of the skin, exposure to UV can cause the scars to darken and increase their prominence, and fibrous tissue in the scar may be silvery in color.

There are several types of treatments that have been described to prevent and to treat acne, however, it is the scarring that is left behind which may be difficult to manage and tougher to control. For example, common treatments for the skin include using topical retinoids to fade brown, red or purple discoloration left behind by acne, laser treatments, dermabrasion and punch grafting. As such, there is a need to treat unsightly scarring and improve its appearance. There is also a desire to reduce the depth, affected area and volume of depressions that may be caused by acne.

### SUMMARY

The invention is defined in the claims.

The present disclosure generally relates to compositions that comprise tropoelastin and methods of using such compositions for the treatment of acne scars (e.g., fibrotic acne scars). The methods may comprise administering a composition that comprises tropoelastin to an area of skin having an acne scar. In an embodiment, the methods comprise treating an area of skin having an acne scar to improve color and/or appearance, and/or reduce depth, affected area, and/or volume of the acne scar in an area of skin of a patient in need thereof. The treatment methods disclosed herein may improve color and/or appearance of the acne scar in the area of skin of a patient in need thereof. Additionally or alternatively, the treatment method may reduce depth, affected area and/or volume of the acne scar in the area of skin of a patient in need thereof.

In some embodiments of each or any of the above- or below-mentioned embodiments, the composition comprises between about 1 mg/ml to about 400 mg/ml tropoelastin. In some embodiments of each or any of the above- or below-mentioned embodiments, the composition comprises about 1 mg/ml, about 5 mg/ml, about 10 mg/ml, about 20 mg/ml, about 30 mg/ml, about 40 mg/ml, about 50 mg/ml, about 60 mg/ml, about 70 mg/ml, about 80 mg/ml, about 90 mg/ml, about 100 mg/ml, about 110 mg/ml, about 120 mg/ml, about 130 mg/ml, about 140 mg/ml, about 150 mg/ml, about 160 mg/ml, about 170 mg/ml, about 180 mg/ml, about 190 mg/ml, about 200 mg/ml, about 210 mg/ml, about 220 mg/ml, about 230 mg/ml, about 240 mg/ml, about 250 mg/ml, about 260 mg/ml, about 270 mg/ml, about 280 mg/ml, about 290 mg/ml, about 300 mg/ml, about 310 mg/ml, about 320 mg/ml, about 330 mg/ml, about 340 mg/ml, about 350 mg/ml, about 360 mg/ml, about 370 mg/ml, about 380 mg/ml, about 390 mg/ml or about 400 mg/ml tropoelastin or any amount of tropoelastin in between a range defined by any two aforementioned values.

In some embodiments of each or any of the above- or below-mentioned embodiments, the composition comprises between about 1 mg/ml to about 300 mg/ml tropoelastin. In some embodiments of each or any of the above- or below-mentioned embodiments, the composition comprises between about 1 mg/ml to about 250 mg/ml tropoelastin. In some embodiments of each or any of the above- or below-mentioned embodiments, the composition comprises between about 1 mg/ml to about 200 mg/ml tropoelastin. In some embodiments of each or any of the above- or below-mentioned embodiments, the composition comprises between about 1 mg/ml to about 150 mg/ml tropoelastin. In some embodiments of each or any of the above- or below-mentioned embodiments, the composition comprises between about 1 mg/ml to about 100 mg/ml tropoelastin.

In some embodiments of each or any of the above- or below-mentioned embodiments, the tropoelastin is crosslinked with about 0.1% to about 10% derivatized hyaluronic acid. In some embodiments of each or any of the above- or below-mentioned embodiments, the composition comprises between about 1 mg/ml to about 100 mg/ml tropoelastin crosslinked with about 0.4% to about 1% derivatized hyaluronic acid (HA). In some embodiments of each or any of the above- or below-mentioned embodiments, the composition comprises about 30 mg/ml recombinant human tropoelastin crosslinked with about 0.5% derivatized hyaluronic acid. In some embodiments of each or any of the above- or below-mentioned embodiments, the composition further comprises a buffer (e.g,. phosphate buffered saline).

In some embodiments of each or any of the above- or below-mentioned embodiments, the composition comprising tropoelastin is administered around the scar, such as beneath the scar and/or around edges of the scar.

In some embodiments of each or any of the above- or below-mentioned embodiments, the acne scars are subclassified as ice pick scars, boxcar scars or rolling atrophic scars. In some embodiments of each or any of the above- or below-mentioned embodiments, the acne scar is an ice pick scar. In some embodiments of each or any of the above- or below-mentioned embodiments, the acne scar is a box scar. In some embodiments of each or any of the above- or below-mentioned embodiments, the scar is a rolling atrophic acne scar. In some embodiments of each or any of the above- or below-mentioned embodiments, the scar is a hypertrophic scar. In some embodiments of each or any of the above- or below-mentioned embodiments, the scar comprises fibrotic tissue around the edges or bottom of the scar.

In some embodiments of each or any of the above- or below-mentioned embodiments, the acne scar has a depth of about 0.1 mm to about 5 mm. In some embodiments of each or any of the above- or below-mentioned embodiments, the acne scar has a depth of about 0.1 mm, about 0.5 mm, about 1.0 mm, about 1.5 mm, about 2 mm, about 2.5 mm, about 3.0 mm, about 3.5 mm, about 4.0 mm, about 4.5 mm, about 5 mm, or any depth in a range in between any two aforementioned values.

In some embodiments of each or any of the above- or below-mentioned embodiments, the acne scar has an affected area of about 0.05 mm² to about 400 mm². In some embodiments of each or any of the above- or below-mentioned embodiments, the acne scar has an affected area of about 0.05 mm², about 0.50 mm², 1.0 mm², about 5 mm², about 10 mm², about 15 mm², about 20 mm², about 25 mm², about 30 mm², about 35 mm², about 40 mm², about 45 mm², about 50 mm², about 55 mm², about 60 mm², about 65 mm², about 70 mm², about 75 mm², about 80 mm², about 85 mm², about 90 mm², about 95 mm², about 100 mm², about 125 mm², about 150 mm², about 175 mm², about 200 mm², about 225 mm², about 250 mm², about 275 mm², about 300 mm², about 325 mm², about 350 mm², about 375 mm², or about 400 mm² or any value in a range in between any two aforementioned values.

In some embodiments of each or any of the above- or below-mentioned embodiments, the acne scar has a volume of about 0.01 mm³ to about 2,000 mm³. In some embodiments of each or any of the above- or below-mentioned embodiments, the acne scar has a volume of about 0.01 mm³, 1 mm³, about 10 mm³, about 20 mm³, about 30 mm³, about 40 mm³, about 50 mm³, about 60 mm³, about 70 mm³, about 80 mm³, about 90 mm³, about 100 mm³, about 125 mm³, about 150 mm³, about 175 mm³, about 200 mm³, about 225 mm³, about 250 mm³, about 275 mm³, about 300 mm³, about 325 mm³, about 350 mm³, about 375 mm³, about 400 mm³, about 425 mm³, about 450 mm³, about 475 mm³, about 500 mm³, about 525 mm³, about 550 mm³, about 575 mm³, about 600 mm³, about 625 mm³, about 650 mm³, about 675 mm³, about 700 mm³, about 725 mm³, about 750 mm³, about 775 mm³, about 800 mm³, about 825 mm³, about 850 mm³, about 875 mm³, about 900 mm³, about 925 mm³, about 950 mm³, 975 mm3, about 1,000 mm³, about 1,100 mm³, about 1,200 mm³, about 1,300 mm³, about 1,400 mm³, about 1,500 mm³, about 1,600 mm³, about 1,700 mm³, about 1,800 mm³, about 1,900 mm³, or about 2,000 mm³, or any volume in a range between any two aforementioned values.

In some embodiments of each or any of the above- or below-mentioned embodiments, the method further comprises a step of disrupting fibrotic strands underneath the acne scar. In some embodiments of each or any of the above- or below-mentioned embodiments, the disrupting is performed prior to administration of the composition to the patient in need. In some embodiments of each or any of the above- or below-mentioned embodiments, the step of disrupting the fibrotic strands creates a dermal pocket underneath the acne scar. In some embodiments of each or any of the above- or below-mentioned embodiments, the disrupting is performed with a 18-32G needle, such as an 18G, 21G, 23G, 25G, 27G, 29G or 30G needle. In some embodiments of each or any of the above- or below-mentioned embodiments, the administering comprises injecting the composition into the dermal pocket. In some embodiments of each or any of the above- or below-mentioned embodiments, the composition comprising tropoelastin is also placed around the scar, such as beneath the scar and around the edges of the scar. An even placement of the composition comprising tropoelastin around the scar throughout the dermis surrounding the scar may ensure that the tropoelastin product is around the scar area.

In some embodiments of each or any of the above- or below-mentioned embodiments, the composition is administered as an injection beneath the acne scar. In some embodiments of each or any of the above- or below-mentioned embodiments, the composition is administered in a volume of about 10 µL to about 100 µL per implant/injection. In some embodiments of each or any of the above- or below-mentioned embodiments, the composition is administered in a volume of about 10 µL, about 20 µL, about 30 µL, about 40 µL, about 50 µL, about 60 µL, about 70 µL, about 80 µL, about 90 µL, or about 100 µL or within a range defined by any two aforementioned values. In some embodiments, more than one injection is made into the acne scar and the total administered volume is about 200 µL, about 300 µL, about 400 µL, or about 500 µL.

In some embodiments of each or any of the above- or below-mentioned embodiments, the injection is given using a retrograde linear threading technique in a cross-hatching arrangement to ensure any fibrous strands within the acne scar are disrupted. In some embodiments of each or any of the above- or below-mentioned embodiments, the needle is inserted at an angle of about 30° parallel to the skin, wherein the needle comprises a bevel and the bevel is facing upwards. In some embodiments of each or any of the above- or below-mentioned embodiments, the needle is inserted more than one time to break up the fibrous strands and create the dermal pocket. In some embodiments of each or any of the above- or below-mentioned embodiments, even pressure is applied to inject the composition at the same time as the needle is withdrawn from the dermal pocket.

In some embodiments of each or any of the above- or below-mentioned embodiments, the administering of the composition is repeated, wherein one or more bolus injections into the scar is administered. In some embodiments of each or any of the above- or below-mentioned embodiments, a maximum volume of composition given as a treatment is between about 100 µL to about 5 mL. In some embodiments of each or any of the above- or below-mentioned embodiments, a maximum volume of composition given as a treatment is about 100 µL, about 500 µL, about 1 ml, about 1.5 ml, about 2 ml, about 2.5 ml, about 3 ml, about 3.5 ml, about 4.0 ml, about 4.5 ml, about 5 ml or any amount in between a range defined by any two aforementioned values. In some embodiments of each or any of the above- or below-mentioned embodiments, the maximum volume of composition given as a treatment is between about 100 µL to about 500 µL per square cm. In some embodiments of each or any of the above- or below-mentioned embodiments, the maximum volume of composition given as a treatment is about 100 µL per square cm, about 150 µL per square cm, about 200 µL per square cm, about 250 µL per square cm, about 300 µL per square cm, about 350 µL per square cm, about 400 µL per square cm, about 450 µL per square cm, about 500 µL per square cm or any amount in between a range defined by any two aforementioned values.

In some embodiments of each or any of the above- or below-mentioned embodiments, the patient in need has a skin type on a Fitzpatrick skin type scale of I, II, III, IV, V or VI. In some embodiments of each or any of the above- or below-mentioned embodiments, the scar comprises a grade of 1-5. The scar grade is as described in a Global Scale for Acne Scar Severity (SCAR-S) by Tan et al. 2010 (Journal of Cutaneous Medicine and Surgery, Vol 14, No 4 (July/August).

In some embodiments of each or any of the above- or below-mentioned embodiments, the acne scars are subclassified as ice pick scars, boxcar scars or rolling atrophic scars. In some embodiments of each or any of the above- or below-mentioned embodiments, the acne scar is an ice pick scar. In some embodiments of each or any of the above- or below-mentioned embodiments, the acne scar is a box scar. In some embodiments of each or any of the above- or below-mentioned embodiments, the scar is a rolling atrophic acne scar. In some embodiments of each or any of the above- or below-mentioned embodiments, the scar is a hypertrophic scar. In some embodiments of each or any of the above- or below-mentioned embodiments, the acne scar comprises fibrotic tissue around the edges of the scar and/or bottom of the scar.

In some embodiments of each or any of the above- or below-mentioned embodiments, the acne scar has a depth of about 0.1 mm to about 5 mm. In some embodiments of each or any of the above- or below-mentioned embodiments, the acne scar has a depth of about 0.1 mm, about 0.5 mm, about 1.0 mm, about 1.5 mm, about 2 mm, about 2.5 mm, about 3.0 mm, about 3.5 mm. about 4.0 mm, about 4.5 mm, about 5 mm, or any depth in a range in between any two aforementioned values. In some embodiments of each or any of the above- or below-mentioned embodiments, the acne scar has an affected area of about 0.05 mm² to about 400 mm². In some embodiments of each or any of the above- or below-mentioned embodiments, the acne scar has a volume of about 0.01 mm³ to 2,000 mm³.

In some embodiments of each or any of the above- or below-mentioned embodiments, the acne scar has a volume of about 0.01 mm³, 1 mm³, about 10 mm³, about 20 mm³, about 30 mm³, about 40 mm³, about 50 mm³, about 60 mm³, about 70 mm³, about 80 mm³, about 90 mm³, about 100 mm³, about 125 mm³, about 150 mm³, about 175 mm³, about 200 mm³, about 225 mm³, about 250 mm³, about 275 mm³, about 300 mm³, about 325 mm³, about 350 mm³, about 375 mm³, about 400 mm³, about 425 mm³, about 450 mm³, about 475 mm³, about 500 mm³, about 525 mm³, about 550 mm³, about 575 mm³, about 600 mm³, about 625 mm³, about 650 mm³, about 675 mm³, about 700 mm³, about 725 mm³, about 750 mm³, about 775 mm³, about 800 mm³, about 825 mm³, about 850 mm³, about 875 mm³, about 900 mm³, about 925 mm³, about 950 mm³, 975 mm3, about 1,000 mm³, about 1,100 mm³, about 1,200 mm³, about 1,300 mm³, about 1,400 mm³, about 1,500 mm³, about 1,600 mm³, about 1,700 mm³, about 1,800 mm³, about 1,900 mm³, or about 2,000 mm³, or any volume in a range between any two aforementioned values.

In some embodiments of each or any of the above- or below-mentioned embodiments, the skin comprises a skin color defined by CIE L*a*b* color coordinates, wherein the method further decreases L*. In some embodiments of each or any of the above- or below-mentioned embodiments, the method decreases L* by about 1%, about 5%, about 10%, about 15%, about 20%, about 25% or greater than about 50%, or any amount defined by a range in between any two aforementioned values. In some embodiments of each or any of the above- or below-mentioned embodiments, the method decreases L* greater than about 50%. In some embodiments of each or any of the above- or below-mentioned embodiments, the method further increases a*. In some embodiments of each or any of the above- or below-mentioned embodiments, the method increases a* by about 1%, about 5%, about 10%, about 15%, about 20%, about 25% or greater than about 50%, or any amount defined by a range in between any two aforementioned values. In some embodiments of each or any of the above- or below-mentioned embodiments, the method increases a* by greater than about 50%. In some embodiments of each or any of the above- or below-mentioned embodiments, the scar comprises a silver coloring prior to administering the composition. In some embodiments of each or any of the above- or below-mentioned embodiments, administering the composition decreases the silver coloring and increases red coloring and/or pink coloring of the scar. In some embodiments of any one of each or any of the above- or below-mentioned embodiments, the method decreases L* by about 1%, about 5%, about 10%, about 15%, about 20%, about 25% or greater than about 50%, or any amount defined by a range in between any two aforementioned values and increases a* by about 1%, about 5%, about 10%, about 15%, about 20%, about 25% or greater than about 50%, or any amount defined by a range in between any two aforementioned values. In some embodiments of each or any of the above- or below-mentioned embodiments, the scar comprises a color, wherein the color is different from a natural skin coloring of the patient, wherein administering the composition results in the color of the scar decreasing or fading in color intensity such that the color of the scar integrates into the natural skin coloring of the patient.

In some embodiments of each or any of the above- or below-mentioned embodiments, the acne scar is a facial, back, or a torso scar. In some embodiments of each or any of the above- or below-mentioned embodiments, the depth, affected area and/or volume of the acne scar is reduced following administering of the composition.

In some embodiments of each or any of the above- or below-mentioned embodiments, the method supports the repair of an atrophic scar during the skin remodeling and maturation phase to reduce the appearance of the acne scar.

In some embodiments of each or any of the above- or below-mentioned embodiments, the method provides a reduction in the acne scar area by about 10%, about 20%, about 30%, about 40%, about 50%, about 60%, about 70%, about 80% or greater than about 90% or any amount in between a range described by any two aforementioned values. In some embodiments of each or any of the above- or below-mentioned embodiments, the method provides a reduction in the acne scar area by greater than about 90%. In some embodiments of each or any of the above- or below-mentioned embodiments, the composition provides a reduction in the acne scar area by about 10%, about 20%, about 30%, about 40%, about 50%, about 60%, about 70%, about 80% or greater than about 90% or any amount in between a range described by any two aforementioned values. In some embodiments of each or any of the above- or below-mentioned embodiments, the composition provides a reduction in the acne scar area by greater than about 90%.

In some embodiments of each or any of the above- or below-mentioned embodiments, the method provides a reduction in the acne scar volume by about 10%, about 20%, about 30%, about 40%, about 50%, about 60%, about 70%, about 80% or greater than about 90% or any amount in between a range described by any two aforementioned values. In some embodiments of each or any of the above- or below-mentioned embodiments, the method provides a reduction in the acne scar volume by greater than about 90%. In some embodiments of each or any of the above- or below-mentioned embodiments, the composition provides a reduction in the acne scar volume by about 10%, about 20%, about 30%, about 40%, about 50%, about 60%, about 70%, about 80% or greater than about 90% or any amount in between a range described by any two aforementioned values. In some embodiments of each or any of the above- or below-mentioned embodiments, the composition provides a reduction in the acne scar volume by greater than about 90%.

In some embodiments of each or any of the above- or below-mentioned embodiments, the method provides a reduction in the acne scar depth by about 10%, about 20%, about 30%, about 40%, about 50%, about 60%, about 70%, about 80% or greater than about 90% or any amount in between a range described by any two aforementioned values. In some embodiments of each or any of the above- or below-mentioned embodiments, the method provides a reduction in the acne scar depth by greater than about 90%. In some embodiments of each or any of the above- or below-mentioned embodiments, the composition provides a reduction in the acne scar depth by about 10%, about 20%, about 30%, about 40%, about 50%, about 60%, about 70%, about 80% or greater than about 90% or any amount in between a range described by any two aforementioned values. In some embodiments of each or any of the above- or below-mentioned embodiments, the composition provides a reduction in the acne scar depth by greater than about 90%.

In some embodiments of each or any of the above- or below-mentioned embodiments, wherein the patient in need has a region of acne scarring on the skin, the method provides a reduction in the depression volume and area of skin contours in the region of acne scarring by about 10%, about 20%, about 30%, about 40%, about 50%, about 60%, about 70%, about 80% or greater than about 90% or any amount in between a range described by any two aforementioned values. In some embodiments of each or any of the above- or below-mentioned embodiments, wherein the patient in need has a region of acne scarring on the skin, the method provides a reduction in the depression volume and area of skin contours in the region of acne scarring by greater than about 90%. In some embodiments of each or any of the above- or below-mentioned embodiments, the composition provides a reduction in the depression volume and area of skin contours in the region of acne scarring by about 10%, about 20%, about 30%, about 40%, about 50%, about 60%, about 70%, about 80% or greater than about 90% or any amount in between a range described by any two aforementioned values. In some embodiments of each or any of the above- or below-mentioned embodiments, the composition provides a reduction in the depression volume and area of skin contours in the region of acne scarring by greater than about 90%.

In some embodiments of each or any of the above- or below-mentioned embodiments, an individual may have almost complete removal of the acne scar by the treatments and methods described herein, wherein the acne depth, volume, and area are reduced by greater than about 90%.

In some embodiments of each or any of the above- or below-mentioned embodiments, the method improves skin color. In some embodiments, improving skin color comprises decreasing L* by about 1%, about 5%, about 10%, about 15%, about 20%, about 25% or greater than about 50%, or any amount defined by a range in between any two aforementioned values and/or increasing a* by about 1%, about 5%, about 10%, about 15%, about 20%, about 25% or greater than about 50%, or any amount defined by a range in between any two aforementioned values. In some embodiments of each or any of the above- or below-mentioned embodiments, L* is decreased by greater than about 50%. In some embodiments of each or any of the above- or below-mentioned embodiments, a* is increased by greater than about 50%. In some embodiments of each or any of the above- or below-mentioned embodiments, the composition provides an improvement in skin color.

In some embodiments of each or any of the above- or below-mentioned embodiments, the method and composition increases volume and area of elevations of skin contours in the region of acne scarring by about 10%, about 20%, about 30%, about 40%, about 50%, about 60%, about 70%, about 80% or greater than about 90% or any amount in between a range described by any two aforementioned values. In some embodiments of each or any of the above- or below-mentioned embodiments, volume and area of elevations of skin contours in the region of acne scarring is increased by greater than about 90%.

In some embodiments of each or any of the above- or below-mentioned embodiments, the method and composition levels the skin contours in a treatment area, wherein the treatment area comprises acne scarring or depressions.

In some embodiments of each or any of the above- or below-mentioned embodiments, the method and composition reduces the L* black to white scale in the treatment field as compared to a control treatment. In some embodiments of each or any of the above- or below-mentioned embodiments, the method and composition increases the a* green to red color scale in the composition treatment field as compared to a control treatment field.

The disclosure also provides methods to treat (e.g., restore) skin countours affected by acne scarring. The methods may comprise administering a composition that comprises tropoelastin to an area of skin with skin countours affected by acne scarring. In an embodiment, the methods comprise treating an area of skin with skin countours affected by acne scarring to reduce the acne scar depth, volume, and/or area.

### BRIEF DESCRIPTION OF THE DRAWINGS

Various features of illustrative embodiments of the present disclosure are described below with reference to the drawings. The illustrated embodiments are intended to illustrate, but not to limit, the present disclosure. The drawings contain the following figures.

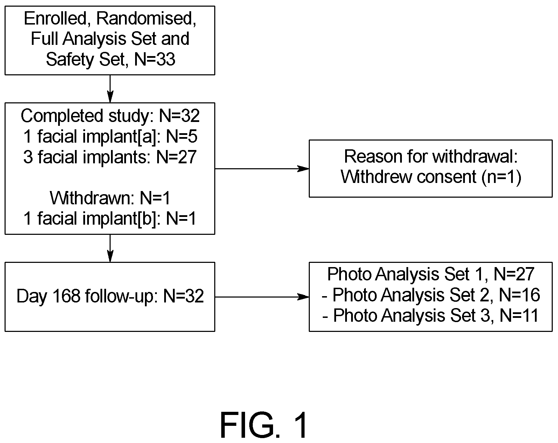

Figure 1 depicts a flow chart of a patient disposition.

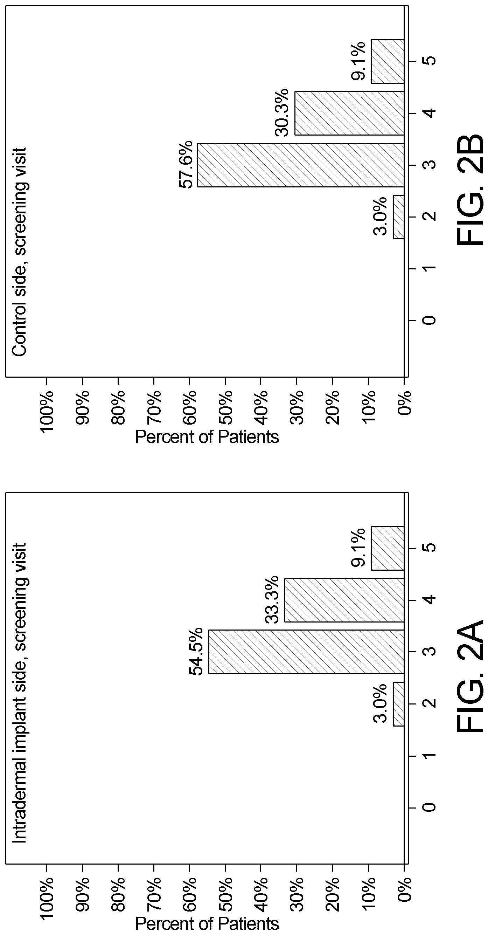

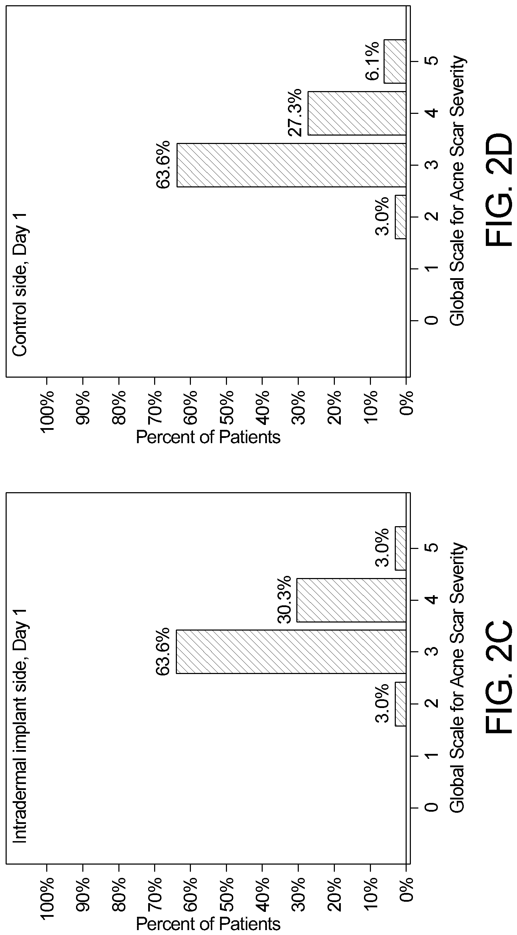

Figures 2A to 2D show the clinical grading of acne scarring at screening and day 1. The grading is based on an acne scarring scale as described in Tan et al. 2010 (Journal of Cutaneous Medicine and Surgery, Vol 14, No 4 (July/August). Scale: 0 = no visible acne scars; 1 = hardly visible acne scars from 2.5 m distance; 2 = easily recognized acne scars, less than half the affected area involved; 3 = easily recognized acne scars, more than half the affected area involved; 4 = easily recognized acne scars, entire area involved; entire area involved with prominent atrophic scars. As shown, is the side with the TE implant side (30mg/ml recombinant human tropoelastin crosslinked with 0.5% derivatized hyaluronic acid) during the screening visit (Figure 2A) and the Saline Control side (Intradermal (i.d.) implants of isotonic saline) (Figure 2B). As shown, is the side with the TE implant during the day 1 (Figure 2C) and the Saline Control side during day 1 (Figure 2D).
Figure 3 shows blind third party review (BTPR) assessment of scars using Global Impression of Change scale (PAS1). Scale: -3 = much worse; -2 = moderately worse; -1 = slightly worse; 0 = no change; 1 = slightly better, 2 = moderately better; 3 = much better.

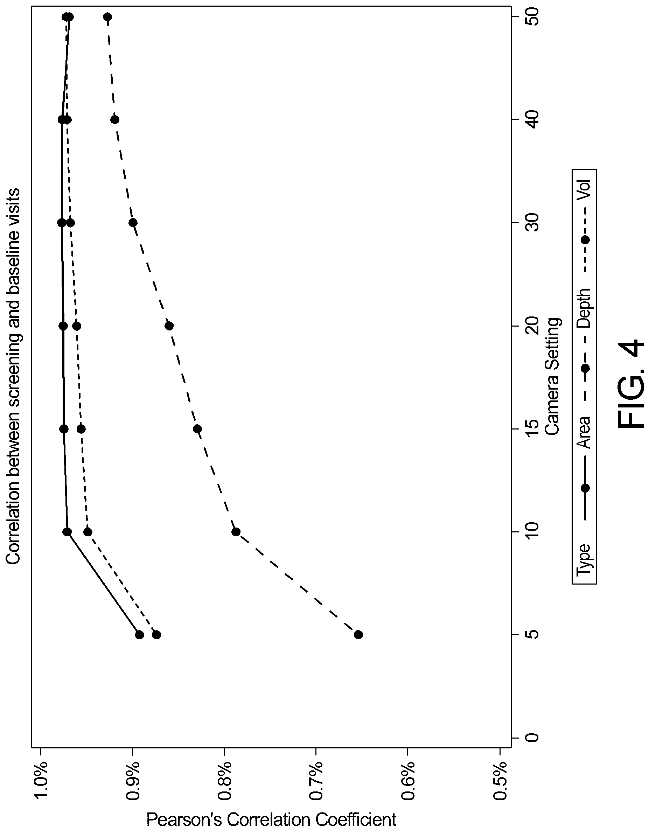

Figure 4 shows the correlation between screening and baseline visits.

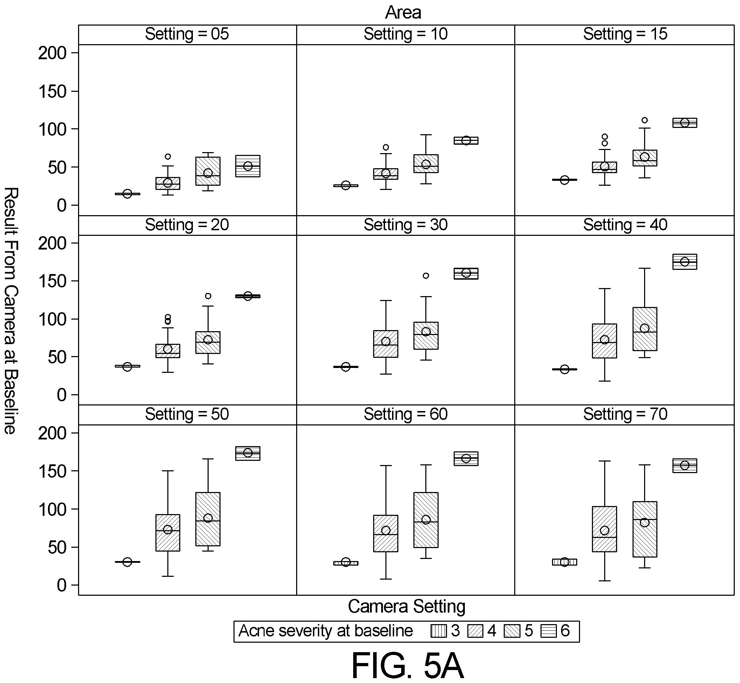

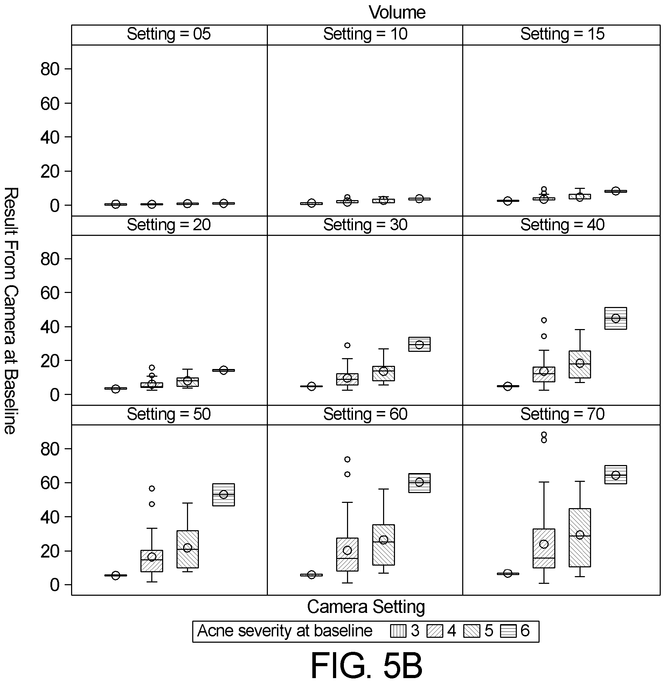

Figures 5A and 5B show results for all camera settings for area and volume, the results from the camera are increasing with increasing severity score and hence suggests the camera is measuring a similar construct.

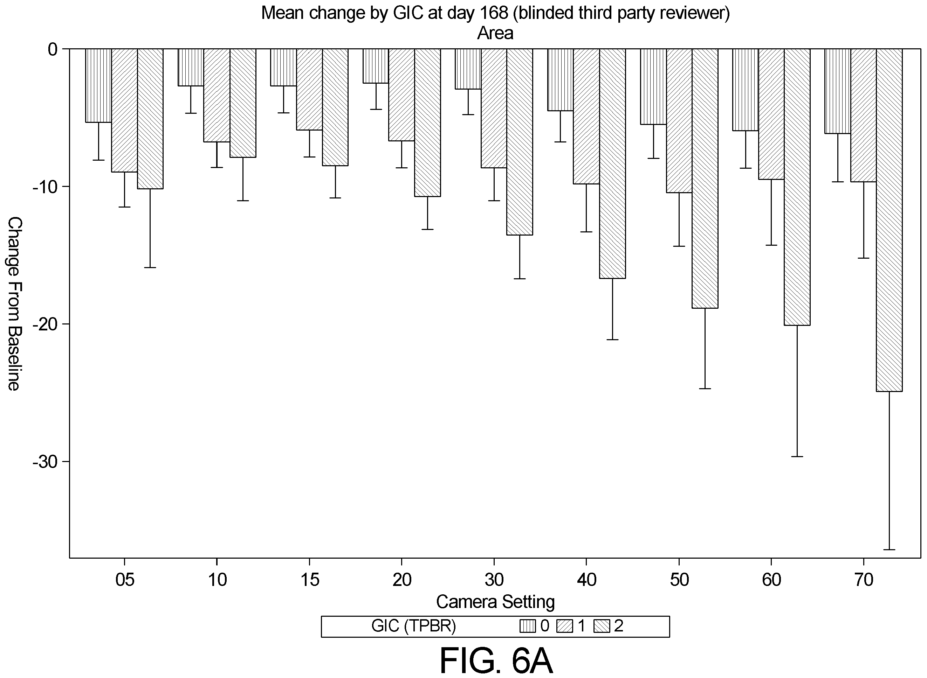

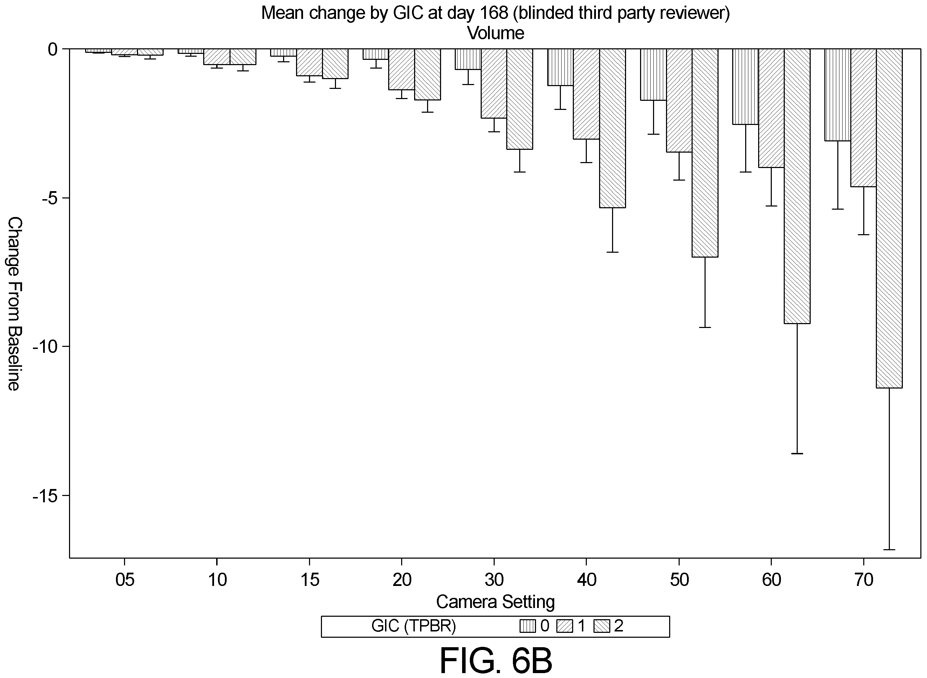

Figures 6A and 6B show the relationship between GIC (BTPR) and results for different camera settings.

### DETAILED DESCRIPTION

Acne is a chronic inflammatory disease that may result from bacterial colonization of pores and hair follicles on the skin of the face, neck, chest and back. The bacterial colonization may be caused by bacteria, such as *Propionibacterium acnes,* for example. Acne may also be caused by increased sebum production, altered keratinization and inflammation. However, sometimes, the triggering of an acne flareup is unclear.

Acne may start at early puberty and into adulthood, during a time where there is increased oil production which may support the growth and colonization of several types of bacteria. Increased oil production may also be caused by hormones, stress and even diet.

Problems associated with acne include soreness, itchiness, pain and may also affect the quality of life. Although there are available treatments for preventing and healing acne, people may be left with the aftermath of their acne, such as unsightly and permanent acne scarring, which can also be highly visible. As acne affects an age group from pre-teens to adults, acne scarring affects pre-teens as well as adults, and can lead to loss of confidence and may lead to low self-esteem.

There are various degrees of scarring that can occur as a result of acne. Scarring may include rolling atrophic, ice-pick scars, boxcar scars and other types of deep depressions in this skin. The depth and extent of acne scarring may vary. Previous treatments have been described such as subcision, punch excision, laser resurfacing, dermabrasion and chemical peels. In some cases, the scars can also lead to discoloration of the skin.

Disclosed herein are methods of treating acne scarring which may help to decrease skin depression, decrease discoloration of the skin and/or even out the contours of the skin.

Unless defined otherwise, all technical and scientific terms used herein have the same meaning as commonly understood by one of ordinary skill in the art to which the invention pertains.

The terms "a," "an," "the" and similar referents used in the context of describing the invention (especially in the context of the following claims) are to be construed to cover both the singular and the plural, unless otherwise indicated herein or clearly contradicted by context.

"About" as used herein when referring to a measurable value is meant to encompass variations of + 20 % or + 10 % , more preferably + 5 % , even more preferably + 1 % , and still more preferably + 0. 1 % from the specified value.

As used herein, except where the context requires otherwise, the term 'comprise' and variations of the term, such as "comprising," "comprises" and "comprised," are not intended to exclude further additives, components, integers or steps.

As used herein, "scarring," "fibrosis," or "fibrotic response" may refer to formation of fibrous (scar) tissue in response to injury or medical intervention.

A "scar," as used herein, may refer to a mark left on the skin or within body tissue where a wound, such as a blemish or acne has healed and fibrous connective tissue has developed. As described herein are scars that may be a result from acne.

The term "tropoelastin" refers to a protein from which elastin is formed. Tropoelastin may be monomeric. Tropoelastin is generally not cross-linked, covalently or otherwise. Tropoelastin may reversibly coacervate. Thus, tropoelastin is distinguished from elastin because elastin consists of covalently cross linked tropoelastin which cannot reversibly coacervate. The tropoelastin may be human tropoelastin. Tropoelastin may be synthetic, for example it may be derived from recombinant expression or other synthesis, or it may be obtained from a natural source such as porcine aorta. As generally known in the art, tropoelastin may exist in the form of a variety of fragments. In some embodiments of each or any of the above- or below-mentioned embodiments, the composition provided in the methods herein comprises monomeric tropoelastin. In some embodiments of each or any of the above- or below-mentioned embodiments, the monomeric tropoelastin is cross-linked to HA. In some embodiments of each or any of the above- or below-mentioned embodiments, the tropoelastin comprises the sequence set forth in any one of SEQ ID NOs: 1-15.

In some embodiments of each or any of the above- or below-mentioned embodiments, the methods of the invention utilize the SHELδ26A tropoelastin analogue (WO 1999/03886) for the various applications described herein including for the compositions that are used in the described methods. The amino acid sequence of SHELδ26A is:

GGVPGAIPGGVPGGVFYPGAGLGALGGGALGPGGKPLKPVPGGLAG
AGLGAGLGAFPAVTFPGALVPGGVADAAAAYKAAKAGAGLGGVPGVGGLGVSAGAV
VPQPGAGVKPGKVPGVGLPGVYPGGVLPGARFPGVGVLPGVPTGAGVKPKAPGVGGA
FAGIPGVGPFGGPQPGVPLGYPIKAPKLPGGYGLPYTTGKLPYGYGPGGVAGAAGKAG
YPTGTGVGPQAAAAAAAKAAAKFGAGAAGVLPGVGGAGVPGVPGAIPGIGGIAGVGTP
AAAAAAAAAKAAKYGAAAGLVPGGPGFGPGVVGVPGAGVPGVGVPGAGIPVVPGA
GIPGAAVPGVVSPEAAAKAAAKAAKYGARPGVGVGGIPTYGVGAGGFPGFGVGVGGIP
GVAGVPSVGGVPGVGGVPGVGISPEAQAAAAAKAAKYGVGTPAAAAAKAAAKAAQF
GLVPGVGVAPGVGVAPGVGVAPGVGLAPGVGVAPGVGVAPGVGVAPGIGPGGVAAA
AKSAAKVAAKAQLRAAAGLGAGIPGLGVGVGVPGLGVGAGVPGLGVGAGVPGFGAV
PGALAAAKAAKYGAAVPGVLGGLGALGGVGIPGGVVGAGPAAAAAAAKAAAKAAQF
GLVGAAGLGGLGVGGLGVPGVGGLGGIPPAAAAKAAKYGAAGLGGVLGGAGQFPLG
GVAARPGFGLSPIFPGGACLGKACGRKRK (SEQ ID NO: 1).

In some embodiments of each or any of the above- or below-mentioned embodiments, the tropoelastin isoform is the SHEL isoform (WO 1994/14958): 
SMGGVPGAIPGGVPGGVFYPGAGLGALGGGALGPGGKPLKPVPGGLAGAGLGAGLGA
FPAVTFPGALVPGGVADAAAAYKAAKAGAGLGGVPGVGGLGVSAGAVVPQPGAGVK
PGKVPGVGLPGVYPGGVLPGARFPGVGVLPGVPTGAGVKPKAPGVGGAFAGIPGVGPF
GGPQPGVPLGYPIKAPKLPGGYGLPYTTGKLPYGYGPGGVAGAAGKAGYPTGTGVGPQ
AAAAAAAKAAAKFGAGAAGVLPGVGGAGVPGVPGAIPGIGGIAGVGTPAAAAAAAA
AKAAKYGAAAGLVPGGPGFGPGVVGVPGAGVPGVGVPGAGIPVVPGAGIPGAAVPGV
VSPEAAAKAAAKAAKYGARPGVGVGGIPTYGVGAGGFPGFGVGVGGIPGVAGVPSVG
GVPGVGGVPGVGISPEAQAAAAKAAKYGVGTPAAAAAKAAAKAAQFGLVPGVGVA
PGVGVAPGVGVAPGVGLAPGVGVAPGVGVAPGVGVAPGIGPGGVAAAAKSAAKVAA
KAQLRAAAGLGAGIPGLGVGVGVPGLGVGAGVPGLGVGAGVPGFGAGADEGVRRSLS
PELREGDPSSSQHLPSTPSSPRVPGALAAAKAAKYGAAVPGVLGGLGALGGVGIPGGVV
GAGPAAAAAAAKAAAKAAQFGLVGAAGLGGLGVGGLGVPGVGGLGGIPPAAAAKAA
KYGAAGLGGVLGGAGQFPLGGVAARPGFGLSPIFPGGACLGKACGRKRK (SEQ IS NO:
 2) or a protease resistant derivative of the SHEL or SHELδ26A isoforms (WO 2000/04043; included by reference in its entirety herein). As described in WO 2000/04043, the protein sequences of tropoelastin described may have a mutated sequence that leads to a reduced or eliminated susceptibility to digestion by proteolysis. Without being limiting, the tropoelastin amino acid sequence has a reduced or eliminated susceptibility to serine proteases, thrombin, kallikrein, metalloproteases, gelatinase A, gelatinase B, serum proteins, trypsin or elastase, for example In some embodiments of each or any of the above- or below-mentioned embodiments, the tropoelastin comprises a sequence set forth in SEQ ID NO: 3 (SHELδ26A isoform): 
GGVPGAIPGGVPGGVFYPGAGLGALGGGALGPGGKPLKPVPGGLAGAGLGAGLGAFPA
VTFPGALVPGGVADAAAAYKAAKAGAGLGGVPGVGGLGVSAGAVVPQPGAGVKPGK
VPGVGLPGVYPGGVLPGARFPGVGVLPGVPTGAGVKPKAPGVGGAFAGIPGVGPFGGP
QPGVPLGYPIKAPKLPGGYGLPYTTGKLPYGYGPGGVAGAAGKAGYPTGTGVGPQAAA
AAAAKAAAKFGAGAAGVLPGVGGAGVPGVPGAIPGIGGIAGVGTPAAAAAAAAAKA
AKYGAAAGLVPGGPGFGPGVVGVPGAGVPGVGVPGAGIPVVPGAGIPGAAVPGVVSPE
AAAKAAAKAAKYGARPGVGVGGIPTYGVGAGGFPGFGVGVGGIPGVAGVPSVGGVPG
VGGVPGVGISPEAQAAAAKAAKYGVGTPAAAAKAAAKAAQFGLVPGVGVAPGVG
VAPGVGVAPGVGLAPGVGVAPGVGVAPGVGVAPGIGPGGVAAAAKSAAKVAAKAQL
 RAAAGLGAGIPGLGVGVGVPGLGVGAGVPGLGVGAGVPGFGAVPGALAAAKAAKYG AAVPGVLGGLGALGGVGIPGGVVGAGPAAAAAAAKAAAKAAQFGLVGAAGLGGLGV GGLGVPGVGGLGGIPPAAAAKAAKYGAAGLGGVLGGAGQFPLGGVAARPGFGLSPIFP GGACLGKACGRKRK (SEQ ID NO: 3). In some embodiments, the tropoelastin comprises a sequence set forth below (SHELδmod isoform):

GGVPGAVPGGVPGGVFYPGAGFGAVPGGVADAAAAYKAAKAGAGLGGVPGVGGLGV
SAGAVVPQPGAGVKPGKVPGVGLPGVYPGFGAVPGARFPGVGVLPGVPTGAGVKPKA
PGVGGAFAGIPGVGPFGGPQPGVPLGYPIKAPKLPGGYGLPYTTGKLPYGYGPGGVAGA
AGKAGYPTGTGVGPQAAAAAAAKAAAKFGAGAAGFGAVPGVGGAGVPGVPGAIPGIG
GIAGVGTPAAAAAAAAAAKAAKYGAAAGLVPGGPGFGPGVVGVPGFGAVPGVGVPG
AGIPVVPGAGIPGAAGFGAVSPEAAAKAAAKAAKYGARPGVGVGGIPTYGVGAGGFPG
FGVGVGGIPGVAGVPSVGGVPGVGGVPGVGISPEAQAAAAAKAAKYGVGTPAAAAAK
AAAKAAQFGLVPGVGVAPGVGVAPGVGVAPGVGLAPGVGVAPGVGVAPGVGVAPGI
GPGGVAAAAKSAAKVAAKAQLRAAAGLGAGIPGLGVGVGVPGLGVGAGVPGLGVGA
GVPGFGAVPGALAAAKAAKYGAVPGVLGGLGALGGVGIPGGVVGAGPAAAAAAAKA
AAKAAQFGLVGAAGLGGLGVGGLGVPGVGGLGGIPPAAAAKAAKYGAAGLGGVLGG
AGQFPLGGVAARPGFGLSPIFPGGACLGKACGRKRK (SEQ ID NO: 4).

Tropoelastin analogues generally have a sequence that is homologous to a human tropoelastin sequence. Percentage identity between a pair of sequences may be calculated by the algorithm implemented in the BESTFIT computer program. Another algorithm that calculates sequence divergence has been adapted for rapid database searching and implemented in the BLAST computer program. In comparison to the human sequence, the tropoelastin polypeptide sequence may be about 60% identical at the amino acid level, 70% or more identical at the amino acid level, 80% or more identical at the amino acid level, 90% or more identical at the amino acid level, 95% or more identical at the amino acid level, 97% or more identical at the amino acid level, or greater than 99% identical at the amino acid level.

Conservative amino acid substitutions (e.g., Glu/Asp, Val/lle, Ser/Thr, Arg/Lys, Gln/Asn) may also be considered when making comparisons because the chemical similarity of these pairs of amino acid residues are expected to result in functional equivalency in many cases. Amino acid substitutions that are expected to conserve the biological function of the polypeptide would conserve chemical attributes of the substituted amino acid residues such as hydrophobicity, hydrophilicity, side-chain charge, or size.

Recombinant forms of tropoelastin can be produced as shown in WO 1999/03886. These sequences include: 
SMGGVPGAIPGGVPGGVFYPGAGLGALGGGALGPGGKPLKPVPGGLAGAGLGAGLGA
FPAVTFPGALVPGGVADAAAAYKAAKAGAGLGGVPGVGGLGVSAGAVVPQPGAGVK
PGKVPGVGLPGVYPGGVLPGARFPGVGVLPGVPTGAGVKPKAPGVGGAFAGIPGVGPF
GGPQPGVPLGYPIKAPKLPGGYGLPYTTGKLPYGYGPGGVAGAAGKAGYPTGTGVGPQ
AAAAAAAKAAAKFGAGAAGVLPGVGGAGVPGVPGAIPGIGGIAGVGTPAAAAAAAA
AKAAKYGAAAGLVPGGPGFGPGVVGVPGAGVPGVGVPGAGIPVVPGAGIPGAAVPGV
VSPEAAAKAAAKAAKYGARPGVGVGGIPTYGVGAGGFPGFGVGVGGIPGVAGVPSVG
GVPGVGGVPGVGISPEAQAAAAAKAAKYGVGTPAAAAAKAAAKAAQFGLVPGVGVA
PGVGVAPGVGVAPGVGLAPGVGVAPGVGVAPGVGVAPGIGPGGVAAAAKSAAKVAA
KAQLRAAAGLGAGIPGLGVGVGVPGLGVGAGVPGLGVGAGVPGFGAGADEGVRRSLS
PELREGDPSSSQHLPSTPSSPRVPGALAAAKAAKYGAAVPGVLGGLGALGVGIPGGVVG
AGPAAAAAAAKAAAKAAQFGLVGAAGLGGLGVGGLGVPGVGGLGGIPPAAAAKAAK
YGAAGLGGVLGGAGQFPLGGVAARPGFGLSPIFPGGACLGKACGRKRK (SEQ ID NO:
5);:
 
GGVPGAIPGGVPGGVFYPGAGLGALGGGALGPGGKPLKPVPGGLAGAGLGAGLGAFPA
VTFPGALVPGGVADAAAAYKAAKAGAGLGGVPGVGGLGVSAGAVVPQPGAGVKPGK
VPGVGLPGVYPGGVLPGARFPGVGVLPGVPTGAGVKPKAPGVGGAFAGIPGVGPFGGP
QPGVPLGYPIKAPKLPGGYGLPYTTGKLPYGYGPGGVAGAAGKAGYPTGTGVGPQAAA
AAAAKAAAKFGAGAAGVLPGVGGAGVPGVPGAIPGIGGIAGVGTPAAAAAAAAAKA
AKYGAAAGLVPGGPGFGPGVVGVPGAGVPGVGVPGAGIPVVPGAGIPGAAVPGVVSPE
AAAKAAAKAAKYGARPGVGVGGIPTYGVGAGGFPGFGVGVGGIPGVAGVPSVGGVPG
VGGVPGVGISPEAQAAAAAKAAKYGVGTPAAAAAKAAAKAAQFGLVPGVGVAPGVG
VAPGVGVAPGVGLAPGVGVAPGVGVAPGVGVAPGIGPGGVAAAAKSAAKVAAKAQL
RAAAGLGAGIPGLGVGVGVPGLGVGAGVPGLGVGAGVPGFGAVPGALAAAKAAKYG
AAVPGVLGGLGALGGVGIPGGVVGAGPAAAAAAAKAAAKAAQFGLVGAAGLGGLGV
GGLGVPGVGGLGGIPPAAAAKAAKYGAAGLGGVLGGAGQFPLGGVAARPGFGLSPIFP
GGACLGKACGRKRK (SEQ ID NO: 6);
 
MGGVPGAVPGGVPGGVFYPGAGFGAVPGGVADAAAAYKAAKAGAGLGGVPGVGGL
GVSAGAVVPQPGAGVKPGKVPGVGLPGVYPGFGAVPGARFPGVGVLPGVPTGAGVKP
KAPGVGGAFAGIPGVGPFGGPQPGVPLGYPIKAPKLPGGYGLPYTTGKLPYGYGPGGVA
AAGKAGYPTGTGVGPQAAAAAAAKAAAKFGAGAAGFGAVPGVGGAGVPGVPGAIPGI
GGIAGVGTPAAAAAAAAAKAAKYGAAAGLVPGGPGFGPGVVGVPGFGAVPGVGVP
GAGIPVVPGAGIPGAAGFGAVSPEAAAKAAAKAAKYGARPGVGVGGIPTYGVGAGFFP
GFGVGVGGIPGVAGVPSVGGVPGVGGVPGVGISPEAQAAAAKAAKYGVGTPAAAAA
KAAAKAAQFGLVPGVGVAPGVGVAPGVGVAPGVGLAPGVGVAPGVGVAPGVGVAPG
IGPGGVAAAAKSAAKVAAKAQLRAAAGLGAGIPGLGVGVGVPGLGVGAGVPGLGVGA
GVPGFGAVPGALAAAKAAKYGAVPGVLGGLGALGGVGIPGGVVGAGPAAAAAAKA
AAKAAQFGLVGAAGLGGLGVGGLGVPGVGGLGGIPPAAAAKAAKYGAAGLGGVLGG
AGQFPLGGVAARPGFGLSPIFPGGACLGKACGRKRK (SEQ ID NO: 7);
SAMGGVPGALAAAKAAKYGAAVPGVLGGLGALGGVGIPGGVVGAGPAAAAAAKAA
AKAAQFGLVGAAGLGGLGVGGLGVPGVGGLGGIPPAAAAKAAKYGAAGLGGVLGGA
GQFPLGGVAARPGFGLSPIFPGGACLGKACGRKRK (SEQ ID NO: 8);
SAMGALVGLGVPGLGVGAGVPGFGAGADEGVRRSLSPELREGDPSSSQHLPSTPSSPRV
PGALAAAKAAKYGAAVPGVLGGLGALGGVGIPGGVVGAGPAAAAAAKAAAKAAQF
GLVGAAGLGGLGVGGLGVPGVGGLGGIPPAAAAKAAKYGAAGLGGVLGGAGQFPLG
GVAARPGFGLSPIFPGGACLGKACGRKRK (SEQ ID NO: 9);
GIPPAAAAKAAKYGAAGLGGVLGGAGQFPLGGVAARPGFGLSPIFPGGACLGKACGRK
RK (SEQ ID NO: 10);
GAAGLGGVLGGAGQFPLGGVAARPGFGLSPIFPGGACLGKACGRKRK (SEQ ID NO:
11); GADEGVRRSLSPELREGDPSSSQHLPSTPSSPRV (SEQ ID NO: 12);
GADEGVRRSLSPELREGDPSSSQHLPSTPSSPRF (SEQ ID NO: 13);
AAAGLGAGIPGLGVGVGVPGLGVGAGVPGLGVGAGVPGFGAGADEGVRRSLSPELRE
GDPSSSQHLPSTPSSPRVPGALAAAKAAKYGAAVPGVLGGLGALGGVGIPGGVVGAGP
AAAAAAKAAAKAAQFGLVGAAGLGGLGVGGLGVPGVGGLGGIPPAAAAKAAKYGA
AGLGGVLGGAGQFPLGGVAARPGFGLSPIFPGGACLGKACGRKRK (SEQ ID NO: 14);
 and

AAAGLGAGIPGLGVGVGVPGLGVGAGVPGLGVGAGVPGFGAVPGALAAAKAAKYGA
AVPGVLGGLGALGGVGIPGGVVGAGPAAAAAAAKAAAKAAQFGLVGAAGLGGLGVG

GLGVPGVGGLGGIPPAAAAKAAKYGAAGLGGVLGGAGQFPLGGVAARPGFGLSPIFPG
GACLGKACGRKRK (SEQ ID NO: 15).

Tropoelastin may be utilized in a form in which it is linked (e.g., covalently linked) to another molecule such as a biopolymer such as hyaluronic acid. In some embodiments, the tropoelastin is cross-linked to hyaluronic acid. In some embodiments, the composition comprises monomeric tropoelastin. In some embodiments of each or any of the above- or below-mentioned embodiments, the tropoelastin comprises the amino acid sequence set forth in any one of SEQ ID Nos: 1-15.

It is particularly preferred that where tropoelastin is linked to another molecule, the linkage does not impede or limit the biological properties of an unlinked form of tropoelastin.

The purpose of linking tropoelastin with another molecule is typically to enable tropoelastin to be localized to a region and to minimize the likelihood of the tropoelastin diffusing or otherwise migrating from that region.

In some embodiments of each or any of the above- or below-mentioned embodiments, the tropoelastin has a specified degree of purity with respect to the amount of tropoelastin in the composition, as compared with amounts of other proteins or molecules in the composition. In one embodiment, the tropoelastin is in a composition that has at least 75% purity, preferably 85% purity, more preferably more than 90% or 95% purity. Fragments of tropoelastin, i.e., truncated forms of a tropoelastin isoform that arise unintentionally through tropoelastin manufacture may be regarded as an impurity in this context.

It will further be understood that in certain embodiments the tropoelastin may be provided in the form of a composition that consists of or consists essentially of tropoelastin, preferably a full-length isoform of tropoelastin. In some embodiments of each or any of the above- or below-mentioned embodiments, the tropoelastin will be at least about 65% of the length of the relevant tropoelastin isoform, more than about 80% of the full length, more than about 90% or more than about 95% of the full length.

The term "hyaluronic acid" or "HA" may include hyaluronic acid and any of its hyaluronate salts, including, for example, sodium hyaluronate (the sodium salt), potassium hyaluronate, magnesium hyaluronate, and calcium hyaluronate. Hyaluronic acid from a variety of sources may be used herein. For example, hyaluronic acid may be extracted from animal tissues, harvested as a product of bacterial fermentation, or produced in commercial quantities by bioprocess technology. In some embodiments, the composition provided in the methods herein comprises monomeric tropoelastin. In some embodiments, the monomeric tropoelastin is cross-linked to HA. In some embodiments of each or any of the above- or below-mentioned embodiments, the tropoelastin comprises the sequence set forth in any one of SEQ ID NOs: 1-15. In some embodiments of each or any of the above- or below-mentioned embodiments, tropoelastin comprises the sequence set forth in SEQ ID NO: 1.

In some embodiments of each or any of the above- or below-mentioned embodiments, the composition comprises between 1-100 mg/ml tropoelastin crosslinked with about 0.1% to about 10% derivatized hyaluronic acid (HA). In some embodiments of each or any of the above- or below-mentioned embodiments, the composition comprises between 1-100 mg/ml tropoelastin crosslinked with about 0.4% to about 1% derivatized hyaluronic acid (HA). In some embodiments of each or any of the above- or below-mentioned embodiments, the tropoelastin is crosslinked with about 0.4%, about 0.5%, about 0.6%, about 0.8%, about 0.9% or about 1% derivatized hyaluronic acid (HA). In some embodiments, the composition comprises 30 mg/ml recombinant human tropoelastin crosslinked with 0.5% derivatized hyaluronic acid. In some embodiments of each or any of the above- or below-mentioned embodiments, the composition further comprises a buffer, wherein the buffer is phosphate buffered saline. In some embodiments, the composition comprises derivatized HA or underivatized HA, to control the extent to which the HA crosslinks with itself and/or the monomeric protein. In some embodiments of each or any of the above- or below-mentioned embodiments, the derivatized HA cross-links with the TE. In some embodiments, the tropoelastin is monomeric. In some embodiments of each or any of the above- or below-mentioned embodiments, the monomeric tropoelastin is released from the composition.

In some embodiments of each or any of the above- or below-mentioned embodiments, the HA may comprise, at least one linkable moiety, such as at least one crosslinkable moiety, for example, a carboxyl group, a hydroxyl group, an amine, a thiol, an alcohol, an alkene, an alkyne, a cyano group, or an azide, and/or modifications, derivatives, or combinations thereof.

In some embodiments of each or any of the above- or below-mentioned embodiments, the HA may comprise, a spacer group, such that the spacer group can link to the same and/or a second molecule, for example, a second biomolecule or biopolymer.

The HA may be in the range of about 25 to about 10000 disaccharide units or residues. In some embodiments of each or any of the above- or below-mentioned embodiments, hyaluronic acid may be used in the range of 25 to 7,500 disaccharide units or residues.

In some embodiments of each or any of the above- or below-mentioned embodiments, the HA may be low or high molecular weight HA. High molecular weight HA (also referred to as "HMW HA") as described herein generally describes a hyaluronic acid having a molecular weight of at least about 1.0 million Daltons (mw ≥ 10⁶ or 1MDa) to about 4.0 MDa. Low molecular weight HA (sometimes herein referred to as "LMW HA") as used herein, generally describes a hyaluronic acid having a molecular weight of less than about 1.0 MDa.

In some embodiments of each or any of the above- or below-mentioned embodiments, the HA may be activated and/or modified with an activating agent, such as EDC or allylglycidyl ether, and/or modifying agent, such as NHS, HOBt or Bromine.

The term "Fitzpatrick scale" is given its plain and ordinary meaning, in view of this paper and without limitation, may refer to a scientific skin type classification. The Fitzpatrick scale has several different types from types 1-6. This scale is used by dermatologists and aesthetic medicine practitioners to determine which treatments are best suited for different skin types. The scale was developed to measure how skin reacts to ultraviolet light such as sun exposure. Additionally, this knowledge can be used by aesthetic doctors and laser technicians to know before administering a laser or another type of treatment. Type 1 - typically has light, ivory skin but when exposed to the sun always burns and peels but never tans. Type 2 - has a light, fair complexion and burns quickly when exposed to the sun and rarely tans. Type 3 - usually has a beige tint to the skin and may burn when exposed to the sun but is capable of tanning. Type 4 - has an olive skin or light brown tone and will not freckle when exposed to the sun. This person rarely gets a sun burn and tans regularly. Type 5 - has a dark brown or black skin tone, rarely gets a sun burn and always tans under sun exposure. Type 6 - has black and is the darkest skin tone. This person never burns and tans quickly when exposed to the sun. This helpful scale may be used by dermatologists to gauge certain skin's reaction to the sun and how to more quickly identify potentially malignant sun spots. Aesthetic doctors may also use the scale to determine the effectiveness of treatments on different skin types. In some embodiments of each or any of the above- or below-mentioned embodiments, the methods for treating acne scars may be used for individuals that may have the skin type classification of any one of types 1-6 on the Fitzpatrick scale.

The term *"intradermal* implant" is an implant that may go underneath the epidermis. Without being limiting, this may be in the area of skin such as the dermis, subdermis or hypodermis layer, for example. In some embodiments of each or any of the above- or below-mentioned embodiments, an implant may be placed intradermally.

**In** some embodiments of each or any of the above- or below-mentioned embodiments, an implant may be placed within the subcutaneous layer. This placement may be in addition to an intradermal placement of an implant.

A "retrograde linear threading technique" is a method for placing threads of implant into a target tissue. Firstly, the needle is inserted. Secondly, the implant is placed by depressing the plunger of the syringe at the same time as the needle is being withdrawn.

"Cross-hatching" is a method of placing threads of implant in a pattern that creates a "mat" within the target tissue. The movement of the needle in the tissue to complete linear threading in a crosshatching arrangement will disrupt fibrous tissue that is in the way or the path of the needle.

"Acne" as described herein, refers to a skin disease that occurs when hair follicles are clogged with dead skin cells and oil from the skin. It may be characterized by blackheads or whiteheads, pimples and oily skin. In some cases, acne may lead to scarring. In some embodiments of each or any of the above- or below-mentioned embodiments, the patient in need has acne as well as acne scars.

"Acne scars" as described herein, may be caused by inflammation within the skin. The acne scars may be due to abnormal healing following the inflammation caused by the acne. "Atrophic acne scars" may be caused by the loss of collagen from the healing response and are the most common cause of acne scarring.

"Ice pick scars," caused by acne may be narrow deep scars that extend into the dermis. Ice pick scar is a type of atrophic acne scar.

"Rolling atrophic acne scar" is an indented scar that heals below the normal layer of skin tissue.

"Boxcar scar" is a type of acne scarring that may appear as indentations into the skin. They are defined by their sharp edges that may move straight down into the skin and are usually a different size from the acne lesion from which they originated. Boxcar scar is a type of atrophic acne scar.

"Hyperpigmentation" is the darkening of skin caused by increase in melanin. Without being limiting, causes of hyperpigmentation include sun damage, inflammation, skin injury and acne. The skin may respond to damage by the excess production of melanin that is produced from melanocytes. In some embodiments of each or any of the above- or below-mentioned embodiments, the scar comprises a silver coloring prior to the administration of the composition. In some embodiments of any one of each or any of the above- or below-mentioned embodiments, administering the composition decreases the silver coloring and increases the dark red or pink coloring of the scar. In some embodiments of any one of each or any of the above- or below-mentioned embodiments, the method decreases L* by 1%, 5%, 10%, 15%, 20%, 25% or greater than 50%, or any amount defined by a range in between any two aforementioned values and increases a* by 1%, 5%, 10%, 15%, 20%, 25% or greater than 50%, or any amount defined by a range in between any two aforementioned values. The methods described herein may also be used to treat acne scarring and to decrease the discoloration of the skin that has been caused by the acne.

The "CIELAB color space" is used to define the skin color and color change that may occur following treatment of the skin with the composition comprising tropoelastin. The CIELAB color space (also known as CIE L*a*b* or sometimes abbreviated as simply "Lab" color space) is a color space defined by the International Commission on Illumination (CIE) in 1976. It expresses color as three values: L* for the lightness from black (0) to white (100), a* from green (-) to red (+), and b* from blue (-) to yellow (+).

### Methods of Treating a Scar

Methods are provided herein for treating an acne scar (e.g., a fibrotic acne scar) in a patent in need thereof including, for example, improving the color and/or appearance of an acne scar. The methods may comprise administering a composition that comprises tropoelastin to an area of skin in the patient having a scar including, for example, administering the compostion adjacent to or directly into or beneath the scar. In another embodiment, the composition comprising tropoelastin is also placed around the scar, such as beneath the scar and around edges of the scar. The depth, affected area and/or volume of the acne scar is reduced following administering of the composition (including as compared to an otherwise identical composition lacking tropoelastin such as a saline control). Additonally or alternatively, the disclosed methods may increase the elevation volume and area of elevations of skin contours in the region of acne scarring (including as compared to an otherwise identical composition lacking tropoelastin such as a saline control).

The methods may further comprise needle disruption of fibrotic elements in the acne scar, such as fibrotic strands or elements (e.g., fibrotic tissue) underneath the scar to cut or release the fibrotic strands that are tethering the surface of the skin deep into a depression. In an embodiment, disruption of the fibrotic elements creates a dermal pocket beneath the acne scar and the composition is administered into the dermal pocket. After disruption of the scar tissue, the dermal pocket may then be filled with the disclosed compositions.

The disruption of a scar may be performed with a 18G-32G needle, such as a 18G, 21G, 23G, 25G, 27G, 29G or 30G needle. Without being limiting, disruption of the fibrotic elements may be performed for scars, such as rolling atrophic, boxcar or icepick scars. The administering comprises injecting the composition into the dermal pocket. The composition is administered as an injection beneath the acne scar. The composition comprising tropoelastin is also placed around the scar, such as beneath the scar and/or around the edges of the scar. An even or uniform placement of the composition comprising tropoelastin around the scar throughout the dermis surrounding the scar may ensure that the tropoelastin product is around the scar area and may enable a more effective treatment. The composition is administered in a volume of about 10 µL to about 100 µL per implant/injection. The injection is given using a retrograde linear threading technique in a cross-hatching arrangement to ensure any fibrous strands within the acne scar are disrupted.

In some embodiments, disruption of fibrous strands are performed for atrophic scars.

Disrupting of the fibrotic strands may be performed using the needle to disrupt strands which may tether the skin to the underlying tissue and contribute to the depression to create a pocket to make space for the tropoelastin composition to be placed under the acne scar, wherein the fibrotic strands are located. Thus, the tropoelastin composition will be available to the skin tissue cells immediately around the scar so they can grow in and around the product which would remodel the fibrotic elements of the scar.

The methods disclosed herein may lead to the remodeling of the fibrotic elements surrounding the scar.

For basic or classic subcision the needle can be as large as 18G, usually 21G or 23G, for example, however a 27G or 30G needle may also be used as it is small enough to allow some subcision but also enables the creation of a pocket or space around the scar and in the dermal skin layer for placement of the product.

The needle is inserted at an angle of about 30° parallel to the skin, wherein the needle comprises a bevel and the bevel is facing upwards. The needle is inserted more than one time to break up the fibrous strands and create the dermal pocket. Even pressure is applied to inject the composition at the same time as the needle is withdrawn from the dermal pocket. The administering of the composition is repeated, wherein one or more bolus injections into the scar is administered.

The methods disclosed herein may advantageously reduce the depth, affected surface area, and/or volume of the acne scar. Indeed, small depressions, as well as large depressions, were shown to improve at 168 days after administration of the compostion (e.g., 30 mg/ml tropoelastin cross-linked with 0.5% dHA) with increased smoothness and/or leveling of skin countours.. Surprisingly, this also led to the repair of atrophic scars during the remodeling and maturation phase to reduce the scars appearance.

The methods disclosed herein may be used to improve the appearance of atrophic acne scars. As described herein, treatments may be administered at days 0, 28 and 56. Such treatments surprisingly led to improved appearance and coloration of the skin in several patients including, patients with rolling, boxcar or icepick type acne scarring. In some embodiments, wherein the coloration of the scar comprises a silver color, the methods disclosed hereindecrease the silver coloring of the scar and/or increase the red coloring or pink coloring of the scar.

The compositions disclosed herein may comprise about 1 mg/ml to about 400 mg/ml tropoelastin (e.g., 1 mg/ml, 5 mg/ml, 10 mg/ml, 20 mg/ml, 30 mg/ml, 40 mg/ml, 50 mg/ml, 60 mg/ml, 70 mg/ml, 80 mg/ml, 90 mg/ml, 100 mg/ml, 110 mg/ml, 120 mg/ml, 130 mg/ml, 140 mg/ml, 150 mg/ml, 160 mg/ml, 170 mg/ml, 180 mg/ml, 190 mg/ml, 200 mg/ml, 210 mg/ml, 220 mg/ml, 230 mg/ml, 240 mg/ml, 250 mg/ml, 260 mg/ml, 270 mg/ml, 280 mg/ml, 290 mg/ml, 300 mg/ml, 310 mg/ml, 320 mg/ml, 330 mg/ml, 340 mg/ml, 350 mg/ml, 360 mg/ml, 370 mg/ml, 380 mg/ml, 390 mg/ml or 400 mg/ml tropoelastin)..

The compositions disclosed herein may also comprise hyaluronic acid (HA). In a further embodiment, the tropoelastin is crosslinked with about 0.1% to about 10% derivatized hyaluronic acid. In yet a further embodiment, the composition comprises about 30 mg/ml human tropoelastin (e.g., recombinant human tropoelastin) crosslinked with about 0.5% derivatized hyaluronic acid. The composition may further comprise a buffer such as phosphate buffered saline.

Also provided herein are pharmaceutical formulations that comprise the disclosed compositons compising tropoelastin.

A maximum volume of the composition disclosed herein that is administered is between about 100 µL to about 5 mL (e.g., between about 100 µL to about 500 µL per square cm).

In an embodiment, the patient in need thereof has a skin type on a Fitzpatrick skin type scale of I, II, III, IV, V, or VI.

The scar treated by the mehods disclosed herein may comprise a grade of any one of 0, 1, 2, 3, 4 or 5 as described in a Global Scale for Acne Scar Severity (SCAR-S) by Tan et al. 2010 (Journal of Cutaneous Medicine and Surgery, Vol 14, No 4 July/ August). The grading is as follows: 0: Clear; No visible scars from acne, 1: Almost clear; Hardly visible scars from 2.5 m away, 2: Mild; Easily recognizable; less than half the affected area (e.g., face, back or chest) involved; 3: Moderate; More than half the affected area (e.g., face, back or chest) involved; 4: Severe; Entire area involved, 5: Very severe; Entire area with prominent atrophic or hypertrophic scars.

In an embodiment, the acne scar may be an ice pick scar, a boxcar scar, a rolling atrophic acne scar, a distensible rolling atrophic acne scar, or a hypertrophic scar. The scar may be on a subject's face, back or torso.

The acne scar treated by the methods disclosed herein may have a depth of about 0.1 mm to about 5 mm (e.g., about 0.1 mm, about 0.5 mm, about 1.0 mm, about 1.5 mm, about 2 mm, about 2.5 mm, about 3.0 mm, about 3.5 mm. about 4.0 mm, about 4.5 mm, about 5 mm, or any depth in a range in between any two aforementioned values).

The acne scar treated by the methods disclosed herein may have an affected area of about 0.05 mm² to about 400 mm².

The acne scar treated by the methods disclosed herein may have a volume of about 0.01 mm³ to 2,000 mm³ (e.g., about 0.01 mm³, about 1 mm³, about 10 mm³, about 20 mm³, about 30 mm³, about 40 mm³, about 50 mm³, about 60 mm³, about 70 mm³, about 80 mm³, about 90 mm³, about 100 mm³, about 125 mm³, about 150 mm³, about 175 mm³, about 200 mm³, about 225 mm³, about 250 mm³, about 275 mm³, about 300 mm³, about 325 mm³, about 350 mm³, about 375 mm³, about 400 mm³, about 425 mm³, about 450 mm³, about 475 mm³, about 500 mm³, about 525 mm³, about 550 mm³, about 575 mm³, about 600 mm³, about 625 mm³, about 650 mm³, about 675 mm³, about 700 mm³, about 725 mm³, about 750 mm³, about 775 v, about 800 mm³, about 825 mm³, about 850 mm³, about 875 mm³, about 900 mm³, about 925 mm³, about 950 mm³, 975 mm3, about 1,000 mm³, about 1,100 mm³, about 1,200 mm³, about 1,300 mm³, about 1,400 mm³, about 1,500 mm³, about 1,600 mm³, about 1,700 mm³, about 1,800 mm³, about 1,900 mm³, or about 2,000 mm³, or any volume in a range between any two aforementioned values).

The scar treated by the methods disclosed herein may decrease a skin color defined by CIE L*a*b* color coordinates (e.g., decreases L* by about 1%, about 5%, about 10%, about 15%, about 20%, about 25% or greater than about 50%, or any amount defined by a range in between any two aforementioned values). In a preferred embodiment, the methods disclosed herein decrease L* by greater than about 50%.

The methods disclosed herein may further increase a*, including by about 1%, about 5%, about 10%, about 15%, about 20%, about 25% or greater than about 50%, or any amount defined by a range in between any two aforementioned values. In a preferred embodiment, the methods disclosed herein increase a* by greater than about 50%.

In an embodiment, the scar comprises a color that is different from a natural skin coloring of the patient and the compostions disclosed herein results in the color of the scar decreasing or fading in color intensity such that the color of the scar integrates into the natural skin coloring of the patient.

The compositions disclosed herein may improve the appearance of a scar (e.g., reduce the depth, volume, surface area, and/or color of the scar including, by about 5%, about 10%, about 15%, about 20%, about 25%, about 30%, about 35%, about 40%, about 45%, about 50%, about 55%, about 60%, about 65%, about 70%, about 75%, about 80%, about 85%, about 90%, about 95%, about 100% or more as compared to an otherwise identical composition lacking tropoelastin or a saline solution) within about 1 month, about 2 months, about 3 months, about 4 months, about 5 months, about 6 months, about 7 months, about 8 months, about 9 months, about 10 months, about 11 months, or about 12 months after injection in an area of skin having the scar.

The following examples, sequence listing and figures are provided to aid the understanding of the present invention, the true scope of which is set forth in the appended claims.

### EXAMPLES

### Example 1. Evaluation of the Clinical Efficacy, Persistence, Tissue Compatability and Safety of a TE Implant for the Treatment of Atrophic Rolling Acne Scars

### Study design and randomization

A study was performed to test and evaluate the clinical efficacy, persistence, tissue compatibility and safety of a TE implant (30mg/ml recombinant human tropoelastin crosslinked with 0.5% derivatized hyaluronic acid) following dermal implantation of the treatment of atrophic rolling acne scars.

Thirty-four male and female patients aged between 24 and 55 years participated in the study of the TE implant for treatment of acne scarring (30mg/ml recombinant human tropoelastin crosslinked with 0.5% derivatized hyaluronic acid; "TE implant"). However, one of the 34 patients withdrew consent before treatment. The 33 patients were tested within areas of skin that measured approximately 2 cm x 2 cm around the temples and cheek bones on each side of the face, which each contained a minimum of two moderate to severe rolling, distensible atrophic acne scars. The treatment was administered to each side of the face were as follows:
a) Tropoelastin intradermal (i.d.) implants of 30mg/ml recombinant human tropoelastin crosslinked with 0.5% derivatized hyaluronic acid ("TE implant"); and
b) a Saline Control (Intradermal (i.d.) implants of isotonic saline) ("Saline Control").

Each patient acted as their own control, with the TE implant implanted in either the right or left side of the face, and the Saline Control was implanted in the opposite side of the face. All patients were to receive three treatments of the TE implant and the Saline Control (at Day 0, Day 28 and Day 56). At each treatment visit, the TE implant was administered to the same side as that used at Day 0 (with the Saline Control being administered to the same side as that used at Day 0). Each treatment consisted of multiple, discrete, direct i.d. injections of the TE implant, each of approximately 25-50 µl, into the dermis of the skin in the 2 cm x 2 cm treatment fields. Injections were given using a retrograde linear threading technique in a cross-hatching arrangement to ensure any fibrous strands within the scar were disrupted. In addition, treatment could include one or more bolus injections into the dermis of the rolling atrophic acne scar to ensure optimal scar response. The total maximum volume of the TE implant or the Saline Control given to each treatment field at each treatment time-point was approximately 250 µl and no more than 500 µl.

Reviews of the implant sites were undertaken on Day 14. All patients were followed for safety until Day 168.

The patients were blind as to which side of their face or body the TE implant and the Saline Control were implanted. The investigator was unblinded. The third party clinical rater of the post treatment images at Day 168 compared to pre-treatment images at Day 0 was blinded.

### Study Procedure and follow ups

### Screening

A screening period of up to 28 days prior to initial implantation with the study device was used to determine patient eligibility and baseline parameters. During this time, written, signed and dated informed consent was obtained from each patient. Relevant medical history items were recorded, a physical examination was undertaken, and their vital signs were recorded. Additional examinations undertaken at this time included: 12-lead ECG, breath testing for alcohol use, urine drug testing (covering use of amphetamines, cocaine, opiates, cannabis, barbiturates and benzodiazepines), clinical examination of intended treatment fields, 2- D and 3-D photography of the intended treatment fields, scoring of the intended treatment fields against the Global Scale for Acne Scar Severity (Tan *et al.,* 2010), serum pregnancy testing, blood tests for APTT and PT coagulation, blood and urine sampling for laboratory safety testing, blood testing for Hep B, Hep C, and HIV 1 and 2. Tests were then used to determine eligibility of the patients for the study and eligible patients were asked to return for commencement of the study (Treatment visit 1, day 0).

### Treatment with Investigational Device

At Treatment Visit 1 (Day 0), eligible patients attended for initial device implantation. Prior to implant, the following procedures were undertaken:
- review of eligibility criteria;
- documentation of changes to concomitant medications;
- pre-implant vital signs;
- breath testing for alcohol use;
- urine testing for drugs of abuse;
- blood draws for anti-tropoelastin antibody analysis;
- clinical examination of intended treatment fields;
- baseline 2-D photography of the treatment fields;
- baseline 3-D photography of the treatment fields;
- pre-treatment assessment of each treatment field by the investigator against the Global Scale for Acne Scar Assessment (Tan et al. 2010 (Journal of Cutaneous Medicine and Surgery, Vol 14, No 4 (July/August), 2010;
- topical application of EMLA cream (at least 60 min prior to TE or Saline control implant);
- urine pregnancy tests for women of child-bearing potential;

After these procedures were undertaken, patients were implanted with the study device (TE implant or Saline Control implant) to the treatment fields. Sixty minutes after the TE implant and Saline Control implant, the injection sites were physically examined for any reactions and post-treatment 2-D photographs were taken.

At Day 14, an implant site review was undertaken, with the following procedures performed by clinical staff:
- review of an adverse event (AE) diary card;
- clinical examination of treatment fields;
- 2-D photography of the treatment fields;
- 3-D photography of treatment fields;
- Blood for serum anti-tropoelastin antibody analysis;

The second treatment implant visit was to occur on Day 28±3, and the third treatment implant visit was to occur on 56±3. Similar procedures to those done at Day 0 were followed.

Patients were provided with a diary to record any injection site responses of redness, swelling, itchiness and tenderness between each study visit.

### Follow up

Patients returned to the study site on Day 84±3 for follow-up procedures. The following activities were undertaken:
- documentation of changes to concomitant medications
- documentation of adverse events and clinical examination of treatment fields
- physical examination and vital signs
- breath testing for alcohol use
- urine testing for drugs of abuse
- blood draws for anti-tropoelastin antibody analysis
- 2-D photography of treatment fields
- 3-D photography of treatment fields
- urine pregnancy tests for women of child-bearing potential
- assessment of the treatment fields by the patient and investigator against the Global Impression of Change scale and by the patient against the ACNE-Q scales

An additional follow-up visit occurred on Day 168±7. The following activities were undertaken:
- documentation of changes to concomitant medications
- documentation of adverse events and clinical examination of treatment fields
- physical examination and vital signs
- 12-lead ECG
- breath testing for alcohol use
- urine testing for drugs of abuse
- blood draws for anti-tropoelastin antibody analysis
- 2-D photography of treatment fields
- 3-D photography of treatment fields
- urine pregnancy tests for women of child-bearing potential
- laboratory safety test of blood and urine as at screening
- assessment of the treatment fields by the patient and investigator against the Global Impression of Change scale and by the patient against the ACNE-Q scales

At Day 168 a blinded third-party reviewer also assessed the treatment fields against the Global Impression of Change scale by comparing Day 168 and Day 0 photographs.

### Objectives of the Study

The primary objective of the study was to establish the clinical efficacy of the TE implant in males and females with atrophic acne scars.

The secondary objectives of the study were as follows:
- Evaluation of the histological changes of the TE implant (30mg/ml recombinant human tropoelastin crosslinked with 0.5% derivatized hyaluronic acid) on atrophic acne scars.
- Confirmation of the local and systemic AE profile of the TE implant.

### Safety objectives

The safety objective of the study was to evaluate the safety and tolerability of the TE implant.

### Exploratory objectives

The following exploratory objectives were examined:
- Clinical significance of acne scars were analyzed
- Classification of acne scars analyzed by the Blinded Third-Party Reviewer (BRPR)
- Correlation of 3D camera data with Global Scale for Acne Scar Severity, ACNE-Q Acne Scar Score and BTPR Treatment Scores

### Inclusion criteria

Patients eligible for inclusion in this study had to meet ALL of the following criteria:
1. Female/male patients with matched treatment fields of approximately 2 cm x 2 cm on each side of the face on the temples or cheek bones that each contain a minimum of two rolling atrophic acne scars that are distensible when stretched between one's fingers and that are classified as moderate to severe on the Global Scale for Acne Scar Assessment.
2. Acne scars in areas of otherwise normal healthy skin.
3. Age: 18 - 55 years.
4. Capable of providing voluntary informed consent.
5. Good general health.
6. Female patients who are sexually active will be of non-child bearing potential (i.e., surgically sterilized or postmenopausal), abstain from sexual intercourse, or use a reliable method of contraception (e.g. hormonal contraceptive, condom, IUD) for at least 30 days prior to dosing and during the duration of the study, with the exception of long-term follow-up. During long-term follow-up, female patients of childbearing potential will be advised to use contraception, but it won't be mandatory.
7. Fitzpatrick skin types I, II, III, IV or V.

### Exclusion Criteria

Patients were excluded from the study if any of the following criteria were met:
1. Active ongoing acne lesions on the face (or back and torso for the patients who will also receive treatment in these areas).
2. Current or previous treatment of atrophic acne scars with fillers, lasers, or deep chemical peels which reach the dermis, or any other medical or surgical treatment which in the investigators opinion could reasonably be deemed to impact on the results of the current clinical study.
3. Known hypersensitivity to tropoelastin, hyaluronic acid or any other component of the TE implant (30mg/ml recombinant human tropoelastin crosslinked with 0.5% derivatized hyaluronic acid).
4. Female patients with a positive pregnancy test, women refusing to agree to adequate contraception and pregnancy tests during the study, or women who are planning to become pregnant during the period of the trial.
5. Participation in a clinical trial of a pharmacological agent within 1 month prior to screening.
6. Clinically significant hematology or biochemistry findings at screening.
7. Positive test for hepatitis B, hepatitis C or HIV at screening.
8. Bleeding diathesis, anticoagulant drugs, thrombocytopenia or clinically significant prolonged APTT or PT.
9. Chronic use of aspirin, other non-steroidal anti-inflammatory drugs or other anti-platelet agents.
10. History of keloid formation.
11. History of granulomatous or connective tissue disease.
12. Systemic corticosteroids within last 12 weeks.
13. Currently use topical retinoids, or have used topical retinoids in the past 8 weeks.
14. Diabetes or other metabolic disorders that may interfere with the patient's response to treatment in the opinion of the investigator.
15. Any serious medical condition which in the opinion of the investigator would have a strong possibility of requiring systemic corticosteroid medication.
16. Females who are pregnant or lactating.
17. Previous administration of tropoelastin.
18. A history of anaphylaxis or allergic reactions including any known hypersensitivity to lidocaine.
19. Use of any investigational product on the intended implant site in the previous 12 months.

### Disposition

The 33 patients were screened, enrolled, randomized, and received at least one TE implant during the study. One patient (Patient ID no: 001-120) was enrolled but withdrew consent prior to the Day 0 treatment. Five patients did not have any further implants following the initial treatment visit on Day 0. The remaining 27 patients received all three intended facial treatments. All 33 randomized patients were included in the Full Analysis Set (FAS) and Safety Set (SS) populations for analysis. The 27 patients that received all three intended facial treatments formed the Photo Analysis Set 1 (PAS1).

### Demographic and baseline characteristics

The ages of the patients ranged from 24 to 55 years, with an average age of 38.0 years (SD=8.71). Twenty-two patients were male and 11 were female. Sixteen patients were Caucasian, two were Mediterranean, fourteen were Asian, and one was categorized as 'Other'. All 11 women were of child-bearing potential, and each had negative results from their pregnancy tests performed at the screening visit. Four patients were Fitzpatrick skin type II, 13 patients were skin type III, 12 were skin type IV, and four were skin type V.

### Scar assessment using Global Scale for Acne Scar Assessment

At the screening visit and at Day 0, the patients' acne scarring was graded using the following scale shown in Table 1 to ensure patients met the inclusion criteria for scar severity and ensuring comparable contralateral treatment fields. The scale was developed by Tan et al. 2010 (Journal of Cutaneous Medicine and Surgery, Vol 14, No 4 (July/August). The clinician grading of the acne scarring is shown in Figures 2A-2D.

**Table 1: Acne scar assessment from Tan et al. (Responses are also indicated in Figure 2) Exposure and compliance**

| **Category** | **Score** | **Description** |
|---|---|---|
| Clear | 0 | No visible scars from acne |
| Almost Clear | 1 | Hardly visible scars from 2.5 m away |
| Mild | 2 | Easily recognizable; less than half the affected area involved |
| Moderate | 3 | More than half the affected area involved |
| Severe | 4 | Entire area involved |
| Very Severe | 5 | Entire area with prominent rolling atrophic scars |

As indicated, six patients in the FAS (Full Analysis set) received no or only one facial implant of the TE implant and Saline Control, while the remaining 27 received three facial implants of the TE implant and Saline Control.

For the FAS, the average volume injected per TE implant in a visit was 0.33mL. By comparison, the average control volume injected per implant visit was 0.45mL.

The cumulative TE volume injected across all implant visits ranged from 0.10mL to 1.43 mL. The median cumulative TE volume injected was 0.90mL. The cumulative Saline Control volume injected across all implant visits ranged from 0.05mL to 1.83mL. The median cumulative Saline Control volume injected was 1.31mL.

### Primary efficacy analyses

A 3D photographic analysis was used to evaluate the various characteristics of the treated fields based on three images for each of five scar lesions on each side of the face. One patient only had two images taken and recorded by the site and so the data for this patient was based on those two images only.

The lesion characteristics measured by the photographs were:
- Color L*
- Color a*
- Color b*
- Volume (mm³)
- Area (mm²)
- Depth (mm)

Volume, area and depth were each assessed at a camera setting of 6.0 mm. In order to produce a summary statistic for reporting and analysis, the median value of the three images for each lesion characteristic was used. The sum of median values for the five lesions on each side of the face was then used as the summary score for analysis for the patient/side. An example is shown below:
- Characteristic = Volume; Lesion j score = Median (Image 1 characteristic, Image 2 characteristic, Image 3 characteristic), where j=1 to 5; Summary score = Lesion 1 score + Lesion 2 score + Lesion 3 score + Lesion 4 score + Lesion 5 score.

The change from baseline for the summary score on each side of the face was then calculated to be the summary score for that side of the face at Day 168, minus the summary score for that side of the face at Day 0.

The difference in change from baseline to Day 168between the TE implant treated side and the Saline Control treated side was also calculated.

Simple summary statistics for the summary score and change from baseline in the summary score are provided herein. In general, both the TE implant and Saline Control treatment fields show evidence of improvement from baseline, with the TE implant treatment field showing a larger improvement in comparison with the Saline Control treatment field.

Lesion dimension data was also modelled using regression analysis. The change from baseline was used as the dependent variable, with treated side (TE implant or Saline Control implant) and baseline lesion summary score used as independent (explanatory) variables. Results are provided for the change from baseline to Day 168 in Table 2 (PAS1). As per the simple summary statistics, the model results showed a significant reduction in depth, volume and area for both TE implant and Saline Control implant, with a consistently larger decrease from baseline for depth, volume and area measurements in the TE implant treatment field compared with the Saline Control treatment field.

**Table 2: Regression model estimates for change from baseline to Day 168 in lesion area, volume and depth for PAS1.**

| **Variable** | **Summary** | **TE implant site** | **Saline Control site** | **TE implant-Saline control** |
|---|---|---|---|---|
| ***PAS1*** | | | | |
| Depth (6.0 mm setting) | Estimate | -0.4287 | -0.2424 | -0.1864 |
| | [95% CI] | [-0.5593, -0.2982] | [-0.3730, -0.1118] | [-0.3525, -0.0203] |
| | p-value | p<.001 | p<.001 | p=0.029 |
| Area (6.0 mm setting) | Estimate | -19.100 | -4.0541 | -15.046 |
| | [95% CI] | [-23.763, -14.436] | [-8.7173, 0.6091] | [-21.502, -8.5888] |
| | p-value | p<.001 | p=0.087 | p<.001 |
| Volume (6.0 mm setting) | Estimate | -6.8309 | -2.9008 | -3.9301 |
| | [95% CI] | [-8.9088, -4.7530] | [-4.9787, -0.8229] | [-6.8770, -0.9831] |
| | p-value | p<.001 | p=0.007 | p=0.010 |

**Whole of treatment field (PAN) analysis.** In addition to the measurements undertaken on individual lesions in the treatment field, the following characteristics were also assessed over the whole of the treatment field, in order to determine the impact of the treatment implants on overall contour deformities in the treatment field, hereafter referred to as the PAN analysis:

Color image (as noted earlier color changes across the treatment field mirrored color changes at the individual lesion level)
- Color L* (a scale of black to white)
- Color a* (a scale of green to red)
- Color b* (a scale of blue to yellow)

Depression measurements (using a camera setting of 10.0mm to detect contour changes across the treatment site - note at this setting individual acne scar lesions are not measured):
- Depth of contour depressions across the treatment field (mm)
- Area of contour depressions across the treatment field (mm²)
- Volume of contour depressions across the treatment field (mm³)

Elevation measurements (using a camera setting of 10.0mm to detect contour changes across the treatment site - note at this setting individual acne scar lesions are not measured.
- Height of elevation across the treatment field (mm)
- Area of elevation across the treatment field (mm²)
- Volume of elevation across the treatment field (mm³)

All camera measures for the PAN treatment field are described herein. As with the individual lesion data, scores were provided based on three images for each characteristic. For the purposes of deriving a summary score for each patient/side, the median of the three image scores was used. The change from baseline for each patient/side was then calculated as the median score at Day 168 minus the median score at Day 1. The change from baseline in the color characteristics measured may be impacted by seasonal changes through the duration of the study - as these are facial treatment fields exposed to the sun then the color of the skin will change from the winter to summer seasons and vice versa. Due to the structure and mechanism of the 3D camera system used (the camera hood is placed directly on the skin) the images are taken with a consistent lighting and therefore variable lighting at the time of taking the photograph does not impact on the resulting image. In order to account for seasonal changes in skin color, the difference between the implant treated side and the Saline Control treated side was calculated at both baseline and Day 168. The change in this difference was used as the efficacy measure for color L*, color a* and color b* in the PAN treatment field analysis. Simple summary statistics are presented below (all patients in the safety set with data).

As with the lesion analysis, regression modeling using the change from baseline (or the change from baseline in the difference) as the dependent variable, with treated side (TE implant or Saline Control) and baseline value (or difference in baseline value) as the independent (explanatory) variables. Results from the analysis of change from baseline to Day 168 are shown in the tables below.

The results show a significant reduction in the L* black to white scale and significant increase on the a* green to red color scale in the PAN treatment field for the TE implant compared with the Saline Control. In addition, a significant reduction in the larger contour skin depression volume and area and corresponding increase in elevation volume and area across the PAN treatment field is also seen for TE implant compared to Saline Control.

**Table 3: Regression model estimates for change from baseline to Day 168 in PAN color, depression volume and elevation volume for PAS1.**

| **Variable** | **Summary** | **TE implant site** | **Saline Control site** | **TE implant -Saline Control** |
|---|---|---|---|---|
| ***PAS1*** | | | | |
| Color L* | Estimate | - | - | -1.1126 |
| | [95% CI] | | | [-1.6898, -0.5354] |
| | p-value | | | p<.001 |
| Color a* | Estimate | - | - | 0.7980 |
| | [95% CI] | | | [0.3308, 1.2652] |
| | p-value | | | p=0.002 |
| Color b* | Estimate | - | - | -0.6312 |
| | [95% CI] | | | [-0.9243, -0.3381] |
| | p-value | | | p<.001 |
| Depression depth | Estimate | -0.1945 | -0.1465 | -0.0480 |
| | [95% CI] | [-0.2629, -0.1260] | [-0.2149, -0.0781] | [-0.1450, 0.0490] |
| | p-value | p<.001 | p<.001 | p=0.325 |
| Depression area | Estimate | -50.116 | -10.491 | -39.625 |
| | [95% CI] | [-66.019, -34.212] | [-26.395, 5.4127] | [-56.888, -22.361] |
| | p-value | p<.001 | p=0.190 | p<.001 |
| Depression volume | Estimate | -22.151 | -7.4243 | -14.727 |
| | [95% CI] | [-30.821, -13.480] | [-16.267, 1.4182] | [-26.225, -3.2285] |
| | p-value | p<.001 | p=0.098 | p=0.014 |
| Elevation height | Estimate | -0.0238 | -0.0292 | 0.0054 |
| | [95% CI] | [-0.0840, 0.0363] | [-0.0893, 0.0310] | [-0.0749, 0.0857] |
| | p-value | p=0.430 | p=0.334 | p=0.892 |
| Elevation area | Estimate | 53.7515 | -7.6174 | 61.3689 |
| | [95% CI] | [40.4975, 67.0055] | [-20.871, 5.6367] | [42.5259, 80.2118] |
| | p-value | p<.001 | p=0.254 | p<.001 |
| Elevation volume | Estimate | 23.8158 | -11.585 | 35.4011 |
| | [95% CI] | [14.8356, 32.7960] | [-20.750, -2.4206] | [22.3307, 48.4714] |
| | p-value | p<.001 | p=0.014 | p<.001 |

### Scar assessment using Global Impression of Change scale.

A blinded third-party reviewer (BTPR) was asked to review Day 168 and Day 0 photographs in a blinded manner to assess the change in appearance of the treatment fields against the Global Impression of Change (GIC) scale. For the BTPR, only the treatment fields and immediate surrounding areas of the face were visible in the photographs reviewed, no patient details were provided, and patients were randomized so that no pattern of treatment was discernible. The scale used was as follows.

**Table 4: Scar Assessment Scale**

| Much worse | Moderately worse | Slightly worse | No change | Slightly better | Moderately better | Much better |
|---|---|---|---|---|---|---|
| -3 | -2 | -1 | 0 | 1 | 2 | 3 |

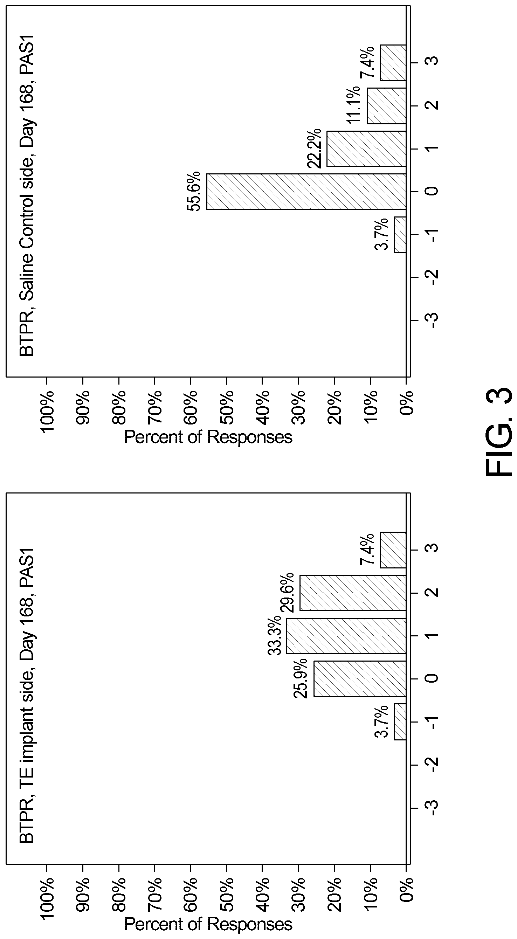

Results of the BRPR assessment for PAS1 are described herein and displayed in Figure 3 (BTPR assessment).

In the PAS1 population, the BTPR noted an improvement at the TE implant site at Day 168 in 70% of treatment fields examined and an improvement at the Saline Control site in 41% of treatment fields examined.

Two additional analyses were undertaken. The first analysis compared the percentage of treatment fields for which the BTPR noted any improvement at the TE implant treatment field against the Saline Control treatment field. The difference in percentages was calculated, and a test of whether the difference in percentages was equal to zero was undertaken. Results are presented in Table 5. There was a 26% difference in the number of treatment fields for which the blinded reviewer noted any improvement at the TE implant site compared with the Saline Control site at Day 168 (p=0.033).

The second analysis used a paired t-test comparison of the GIC score (ranging from -3 to +3) from the TE implant treated side against the Saline Control treated side. These results are presented below in Table 6. The average difference in GIC scores between the tropoelastin implant at Saline Control sites, as assessed by the BTPR at Day 168 was 0.48 (95% CI from -0.04 to 1.00, p=0.068).

**Table 5: Treatment field by blinded third party reviewer (BTPR) noting any improvement via GIC score (Photo Analysis Set 1).**

| **Assessed by:** | **Visit** | **Summary** | **Tropoelastin implant site** | **Saline Control site** | **Implant-Saline Control** | **p-value** |
|---|---|---|---|---|---|---|
| BTPR | Day 168 | n/N | 19/27 | 11/27 | (29.6%) | |
| | | % | (70.4%) | (40.7%) | [1.2%, 54.6%] | |
| | | [95% CI] | [51.3%, 84.3%] | [24.5%, 59.3%] | | 0.033 |

GIC scores are measured from -3 to +3. Scores from +1 to +3 are grouped as improvement, while scores of -3 to 0 are grouped as deterioration/no improvement. This table shows the number of patients assessed as improved (n) over the total number assessed (N). The difference represents the percentage noting improvement at the TE implant site minus the percentage noting improvement at the Saline Control site. The p-value tests for whether the difference in percentages (TE Implant-Saline Control) is zero.

**Table 6: Treatment field for blinded third party reviewer (BTPR) GIC score (Photo Analysis Set 1).**

| **Assessed by:** | **Visit** | **Summary** | **Tropoelastin intradermal Implant site - Saline Control site** |
|---|---|---|---|
| BTPR | Day 168 | Difference[a] | 0.48 |
| | | [95% CI] | [-0.04, 1.00] |
| | | p-value | 0.068 |

GIC scores are measured from -3 to +3. Scores from +1 to +3 are grouped as improvement, while scores of -3 to 0 are grouped as deterioration/no improvement. This table summarizes the paired differences in GIC (TE Implant - Saline Control). A positive difference indicates higher GIC at the TE Implant site compared with Saline Control site. The p-value tests for whether the paired differences (TE Implant-Saline Control) are zero.

### Acne Q questionnaire

At baseline (Day 1) and on Days 84, 168 and 336, patients were asked to complete an Acne-Q questionnaire. The questionnaire consists of 10 questions relating to the patient's perception of how much their acne scars bothered them. Each of the questions were rated on a four-point scale: 1=not at all, 2=a little bit, 3=quite a bit, 4=very much. The ten questions asked are listed below:
1. How much are you bothered by how your acne scars look like from far away?
2. How much are you bothered by the way your acne scars have healed so far (more raised or indented than you would like)?
3. How much are you bothered by the size of your acne scars?
4. How much are you bothered by how your acne scars look in photos?
5. How much are you bothered by the amount of acne scarring you have?
6. How much are you bothered by the difference between the color of your acne scars and your skin color?
7. How much are you bothered by how your acne scars look under a bright light?
8. How much are you bothered by how your acne scars look in the mirror?
9. How much are you bothered by how noticeable your acne scars are?
10. How much are you bothered by how your acne scars look up close?

The scale is described by Klassen et al. (Acne-Q©, A Guide for Researchers and Clinicians, Users Guide Version 1.0, September 2018, McMaster University).

Summary statistics for the acne scar scale for the PAS1 population are shown herein.

The acne scar scale is scored such that a higher total represents a 'better' outcome from the patient perspective. At baseline, there was no statistically significant difference in the patient scoring for both sides of the face in the PAS1 population, with an average score of 36.7 for the TE implant treatment field and 35.4 for the Saline Control treatment field. At Day 168, PAS1 population patients scored their TE implant treatment field (57.4) higher than their Saline Control treatment field (52.0) - the difference, 5.370, was statistically significantly different from zero (p=0.014).

By Day 168, the change from baseline score was statistically significantly higher than zero for both the TE implant treatment field and the Saline Control treatment field. While changes at the TE implant site were generally larger on average in comparison with the Saline Control site, the difference between the two treatment fields was not statistically significantly different from zero (for the PAS1 population, the difference was 4.074, p=0.120).

**Table 7: Acne-Q acne scar score for PAS1 population, by timepoint.**

| **Timepoint** | **TE implant site** | **Saline Control site** | **TE implant -Saline Control** |
|---|---|---|---|
| Day 0 | 36.667 | 35.370 | 1.296 |
| | [30.667, 42.666] | [29.347, 41.394] | [-4.087, 6.680] |
| | | | p=0.625 |
| Day 168 | 57.370 | 52.000 | 5.370 |
| | [50.759, 63.982] | [45.477, 58.523] | [1.181, 9.560] |
| | | | p=0.014 |
| Change from Day 0 to 168 | 20.704 | 16.630 | 4.074 |
| | [14.693, 26.714] | [11.516, 21.743] | [-2.386, 10.534] |
| | p<0.001 | p<0.001 | p=0.120 |

Higher acne-Q scar scores represent a 'better' response from the patient. A positive change from baseline for the acne-Q scar score indicates the patient noted an improvement based on the questionnaire items.

### Secondary objective analyses

The Blinded Third-Party Reviewer was also asked to classify the scars as follows:
- Distensible rolling atrophic acne scar: one that if stretched between the fingers would no longer be visible.
- Boxcar or boxcar like scars: classic boxcar scars and scars that have distinctive fibrotic edges that would remain visible even if stretched between the fingers.
- Icepick scars: classic icepick acne scars.

Overall, at baseline 61 % and 69% of the scars identified as the clinically most significant by the BTPR were classified as Boxcar-type scars on the TE implant and Saline Control treatment sides, respectively. Lower percentages of 37% and 26% of the scars identified as the clinically most significant by the BTPR were classified as Rolling-type scars on the TE implant and Saline Control treatment sides, respectively. This may indicate that Boxcar scars are more likely to present as the clinically most significant scar type. The remaining 2% of scars on the TE implant side and 5% of scars on the Saline Control treatment side were classified as Ice-pick-type.

In order to analyze acne scars by scar type, the same procedures as those used for the primary efficacy analysis were followed. These analyses were limited to volume, depth and affected measurements at the 6.0mm camera lateral size settings.For each lesion identified by the BTPR as one of the three most clinically relevant acne scars, the median value of the three images for each lesion characteristic was used. However, as patients could have differing numbers of lesions identified, the average of the median values for the identified lesions on each side of the face was used as the summary score for analysis for the patient/side, rather than the sum.

The change from baseline for the summary score on each side of the face was then calculated to be the summary score for that side of the face at Day 168, minus the summary score for that side of the face at Day 0.

The difference in change from baseline to Day 168, between the TE implant treated side and the Saline Control treated side was also calculated. As noted previously, not all patients had data for both sides included in this analysis, as in some instances, the BTPR identified one scar type being present within their three most clinically relevant acne scars on one side of the face, but not the other. This should be considered when interpreting the differences in this analysis. Analyses were undertaken separately for Boxcar-type lesions and for Rolling-type lesions.

Simple summary statistics for the summary score and change from baseline in the summary score are provided (PAS1) for Boxcar-type lesions and (PAS1) for Rolling-type lesions.

The BTPR lesion data for Boxcar-type and Rolling acne scars was also modelled using regression analysis as per the primary efficacy analysis. Results are provided for the change from baseline to Day 168 for camera setting 6.0mm for PAS1 Boxcar-type and PAS1 Rolling-type. The model results showed a consistently larger decrease from baseline for depth, volume and area measurements in the TE implant treatment field compared with the Saline Control treatment field.

Results are repeated below for the Boxcar-type lesions in Table 8 (change to Day 168). For Boxcar-type lesions identified by the BTPR, the findings were generally consistent with the primary efficacy analysis findings, although in this case, there were more findings of statistical or close to statistical significance in the difference between the TE implant and Saline Control treatment fields indicating a surprisingly greater effect of the TE compared to Saline Control on scars containing fibrotic tissue.

**Table 8: Regression model estimates for change from baseline to Day 168 in BTPR Boxcar-type lesion area, volume and depth (PAS1 set)**

| **Variable** | **Summary** | **TE implant site** | **Saline Control site** | **TE implant - Saline Control** |
|---|---|---|---|---|
| ***PAS1*** | | | | |
| Depth (setting 6.0) | Estimate | -0.1157 | -0.0403 | -0.0754 |
| | [95% CI] | -0.1479, - 0.0836] | -0.0711, - 2.0096 | -0.1191, - 0.0317 |
| | 1 | p<.001 | p=0.011 | p=0.00 |
| Area (setting 6.0) | Estimate | -5.0980 | -1.1054 | -3.9926 |
| | [95% CI] | [-6.6893, - 3.5067] | -2.6456, 2.4349 | [-5.7645, - 2.2207] |
| | p-value | p<.001 | p=0.15 | p<.001 |
| Volume (setting 6.0) | Estimate | -2.1539 | -0.8870 | -1.2669 |
| | [95% CI] | [-2.8269, - 1.4810] | -1.5378, - 0.2362] | -2.0245, - 0.5093] |
| | p-value | p<.001 | p=0.009 | p=0.002 |

### Correlation of 3D Camera Data with the Global Scale of Acne Scar Severity

As part of the study, a six point Global Scale for Acne Scar Severity (GSASS or SCAR-S, Tan *et al* 2010). was provided by the investigator at Screening and Day 0, for both the left and right side of face treatment fields as an inclusion criteria for the study. The scale was as follows in Table 9.

**Table 9: Global Scale for Acne Scar Severity**

| **Category** | **Score** | **Description** |
|---|---|---|
| Clear | 0 | No visible scars from acne |
| Almost Clear | 1 | Hardly visible scars from 2.5 m away |
| Mild | 2 | Easily recognizable; less than half the affected area |
| Moderate | 3 | More than half the affected area involved |
| Severe | 4 | Entire area involved |
| Very Severe | 5 | Entire area with prominent atrophic scars |

The correlations between the GSASS scores at Day 0 and the photographic measurements of scar lesions from Day ) images were assessed. The majority of GSASS scores at Day 0 were at category 3 or 4 (>90%) of cases, and therefore the spread of data for this variable may not provide an adequate estimate of the true correlation between GSASS and the continuous photographic measurements.

Simple linear regression models were run to estimate the slope of the linear relationship between the photographic measure (dependent variable) and the GSASS (independent variable). The interpretation of the slope estimate is as per usual - that a one-unit change in GSASS (change from category X to category X+1) results in a change in the photographic measure equal to the slope estimate. The expectation is that as GSASS increases, the photographic measure also increases, and so it was expected that the slope estimates will be positive. This is indeed the case, as shown below in Table 10:

**Table 10: Regression estimates from modeling GSASS against photographic data captured at Day 0.**

| **Photo measure** | **Camera setting** | **Slope estimate for GSASS** | **Lower 95% CL** | **Upper 95% CL** | **p-value** | **R-squared** |
|---|---|---|---|---|---|---|
| **Depth** | 6.Umm | 0.357 | 0.076 | U.639 | 0.014 | 0.094 |
| **Area** | 6.Umm | 25.829 | 9.682 | 41.9'/6 | 0.002 | 0.142 |
| **Volume** | 6.0mm | 10.111 | 3.444 | 16.777 | 0.004 | 0.129 |

P-value tests whether the slope estimate is equal to zero and is equivalent to the p-value for the Pearson's correlation coefficient being zero. R-squared is the Pearson's correlation coefficient squared.

In all cases the slope estimate was positive and was significant for each of the photographic measurements by camera setting, with the greatest statistical significance observed from the volume and area measurements (at each camera setting tested). The highest R-squared values were observed for the area and volume for each of the camera software settings, which is not surprising given the GSASS is an assessment of scar visibility from distance. It should be noted that the simple linear regression model does not necessarily account for correlation by patient - that is, each patient is providing both a left-side and a right-side value for analysis, which may not be independent of each other, which is one of the underlying assumptions of ordinary least squares regression. As mentioned previously, the majority of data on the x-axis. (GSASS) is clustered around two values, which may also affect the 'generalization' of the results.

### Correlation of 3D Camera Data with the Third-Party Blinded Reviewer Pre/Post Treatment Scores.

A Blinded Third-Party Reviewer (BTPR) was asked to review Day 168 and Day 0 photographs in a blinded manner to assess the change in appearance of the treatment fields against the GIC scale. For the BTPR, only the treatment fields and immediate surrounding areas of the face were visible in the photographs reviewed. No patient details were provided, and patients were randomized so that no pattern of treatment was discernible. The BTPR was also asked to identify, but not rank, the three most clinically meaningful acne scars based on the baseline photographs provided. As part of the additional exploratory analysis, the correlation between the BTPR GIC score and the change from baseline in the BTPR identified lesions as measured using the 3D camera software was examined.

As per previous data exploration, a series of simple linear regression models were developed with the change from baseline in the photographic measure as the dependent variable and the BTPR GIC score as the independent variable. As a higher GIC score indicates an improvement by visual inspection, the hoped for slope of the line would be negative, indicating a per unit increase in GIC score would result in a drop in measured parameter equal to the slope estimate. This is indeed the case as seen in Table 11 below, for all photographic measurement parameters, at each of the camera settings examined. In each case, the p-value was <0.05. The slope estimates may represent initial estimates of clinically relevant changes in volume, area, and depth.

**Table 11. Regression estimates from modeling BTPR GIC score against change from baseline to Day 168 in BTPR selected lesions from camera data.**

| **Photo measure** | **Camera setting** | **Slope estimate for GIC** | **Lower 95% CL** | **Upper 95% CL** | **p-value** | **R-squared** |
|---|---|---|---|---|---|---|
| **Depth** | 6.Umm | -0.031 | -0.047 | -0.016 | <.001 | 0.205 |
| **Area** | 6.Umm | -1.331 | -2.160 | -0.502 | 0.002 | 0.142 |
| **Volume** | 6.0mm | -0.471 | -0.819 | -0.171 | 0.009 | 0.106 |

pP-value tests whether the slope estimate is equal to zero and is equivalent to the p-value for the Pearson's correlation coefficient being zero. R-squared is the Pearson's correlation coefficient squared.

### Discussion

The TE implants were found to reduce the volume, area and depth of treated facial atrophic acne scars based on 3D photographic analysis and improve their appearance based the blinded third party reviewer feedback .

The TE implants were also found to be safe and tolerable in men and women with facial atrophic acne scars who received implants on three occasions over a period of 56 days.

3D photographic assessment of the facial treatment fields was undertaken at baseline and Day 168, using various camera software settings which measured acne lesion and whole-of-treatment field characteristics of color, depth, area, and volume. The 3D photographic assessments included the assessment of skin characteristics at a level encompassing individual acne scars (camera lateral size settings of 6.0 mm), and at the level of overall skin contours (camera lateral size setting of 10.0mm, where smaller depressions are not detected).

Modeling of the results indicated a statistically significant reduction in scar volumes, area, and depth over time following treatment with TE implant at the measures analyzed (6.0mm setting). Of note, the largest differences for TE implant over the Saline Control were seen at a camera software setting encompassing individual acne scar depressions and overall skin contours.

Facial acne lesions treated with TE implant showed the following changes in the PAS1 population:
- A reduction in acne lesion depth from baseline to Day 168 of -0.43mm using the 6.0mm camera software setting (95%CI from -0.56mm to -0.30mm, p<0.001) - this represented a 21% reduction from baseline. This reduction was statistically significantly larger than the reduction observed at in the acne lesions at the Saline Control site (p=0.029).
- A reduction in acne lesion area from baseline to Day 168 of -19.10mm² using the 6.0mm camera software setting (95%CI from -23.76 mm² to - 14.44mm², p<0.001) - this represented a 24% reduction from baseline. This reduction was statistically significantly larger than the reduction observed at in the acne lesions at the Saline Control site (p<0.001).
- A reduction in acne lesion volume from baseline to Day 168 of -6.83mm³ using the 6.0mm camera software setting (95%CI from -8.91mm³ to -4.75mm³, p<0.001) - this represented a 30% reduction from baseline. This reduction was statistically significantly larger than the reduction observed at in the acne lesions at the Saline Control site (p=0.010).

Modeling also showed a statistically significant difference in the color characteristics and improvement in the skin contours of the whole-of-treatment fields, each in favor of the TE implant sites. The color analysis was conducted using the change from baseline in the difference between the TE implant treatment field and the Saline Control treatment field, due to seasonal impacts on facial skin color. Key results included:
- A change from baseline to Day 168 of -1.11 in the difference between the TE implant and Saline Control sites Color L* characteristic (95%CI from -1.69 to -0.54, p<0.001)
- A change from baseline to Day 168 of +0.79 in the difference between the TE implant and Saline Control sites Color a* characteristic (95%CI from +0.33 to +1.27, p=0.002)
- A change from baseline to Day 168 of -0.63 in the difference between the TE implant and Saline Control sites Color b* characteristic (95%CI from -0.92 to -0.34, p<0.001)

Modeling also showed a statistically significant improvement in the skin contours of the whole-of treatment fields as measured by the larger 10.0mm camera lateral size setting. Key results here from the PAS1 set included:
- A decrease in skin contour depression area from baseline to Day 168 of - 50.12mm³ (95%CI from -66.02 mm³ to -34.21 mm³, p<0.001; representing a 47% decrease from baseline value) at the TE implant treatment field. This was statistically significantly different to the reduction observed at the Saline Control treatment field (p<0.001). (Table 3).
- A reduction in contour depression volume from baseline to Day 168 of - 22.15 mm³ (95%CI from - 30.82mm³ to -13.48 mm³, p<0.001; representing a 50% reduction from baseline value) at the TE implant treatment field. This reduction was statistically significantly larger than the reduction observed at the Saline Control treatment field (p=0.014). (Table 3)
- An increase in skin contour elevation area from baseline to Day 168 of +53.75mm² (95%CI from +40.50mm² to +67.01 mm², p<0.001; representing a 28% increase from baseline value) at the TE implant treatment field. This was statistically significantly different to the reduction observed at the Saline Control treatment field (p<0.001). (Table 3).
- An increase in skin contour elevation volume from baseline to Day 168 of +23.82mm³ (95%CI from +14.84 mm³ to +32.80 mm³, p<0.001; representing a 27% increase from baseline value) at the TE implant treatment field. This was statistically significantly different to the reduction observed at the Saline Control treatment field (p<0.001). (Table 3).
- There was no significant impact of either treatment on either contour depression depth or contour elevation height.

There were also statistically significant differences between the TE implant treatment fields and Saline Control treatment fields (in favor of the TE implant side) using a blinded third-party reviewer based assessments via the Global Impression of Change scale. In the PAS1 population, at Day 168:
- 70% of assessments by the BTPR noted improvement at the TE implant treatment field and 41% noted an improvement at the Saline Control treatment field (difference in percentages, 30%, p=0.033). (Table 5).
- the average difference in GIC assessment by the BTPR was 0.48 in favor of TE implant (p=0.068). (Table 6).

The acne scar scale of the ACNE-Q questionnaire was derived as per the author's instruction (Klassen et al). Based on patient responses, acne scar scale scores were similar for both the left and right side of the face at baseline. By Day 168, the acne scar scale score for the TE implant treated side of the face was significantly higher than that for the Saline Control side (difference=5.438 points, p=0.005. The change from baseline in the acne scar scale was also calculated. At Day 168, both the TE implant site and Saline Control site showed significant improvement from baseline values (p<0.001 for both sides). It should be noted that the ACNE-Q questionnaire is not, at this time, validated for the change from baseline.

In order to evaluate the clinical significance of the measurements derived from the 3D camera data, the correlation of the acne scar sizes as measured by the 3D camera was assessed against the GSASS and Acne-Q scar scales at Day 0; and, the change in acne scar sizes as measured by the 3D camera was assessed against the GIC score provided by the BTPR.

Analysis of the relationship between GSASS assessment done at Day 0 against depth, volume and area measurements taken at Day 0 using the 6.0mm camera setting revealed a correlation with all three measurements as followed
- A one unit step in GSASS was associated with a 0.36mm increase in scar depth across the five lesions measured (95% CI from 0.08mm to 0.64mm, p=0.014). (Table 10).
- A one unit step in GSASS was associated with a 25.83mm² increase in scar area across the five lesions measured (95% CI from 9.68 mm² to 41.98 mm², p=0.002) (Table 10).
- A one unit step in GSASS was associated with a 10.11mm³ increase in scar volume across the five lesions measured (95% CI from 3.44mm³ to 16.78mm³, p=0.004) (Table 10).

The above correlation of the acne scar measurements derived from the 3D camera with all three clinical assessment tools clearly indicates that the 3D camera measurements are clinically meaningful, and such correlations may be useful in calculating sample sizes for future clinical studies. The data can also be used to derive the average step-up in GIC. Using the area measurement of the acne scars at the camera 6.0 setting, the following changes were observed:
Day 168; the TE implant group showed an average decrease from baseline of - 19.0 mm², which represents approximately a 3.7-step change in GIC (based on a step value of -5.2 mm²), while the Saline Control group showed a decrease from baseline of -4.2 mm², which represents approximately a 0.8- step change in GIC.

The analysis found that, for the acne scar area measurement, a camera setting of 6.0mm demonstrated a clinically meaningful difference between the TE implant treatment field and the Saline Control treatment field. Volume measurements taken with the 6.0mmsetting also demonstrated clinically meaningful differences between the TE implant treatment field and Saline Control treatment field.

70% of assessments by the BTPR noted improvement at the TE implant treatment field and 41% noted an improvement at the Saline Control treatment field (difference in percentages, 31%, p=0.012).

Based on patient responses, acne scar scale scores from the ACNE-Q questionnaire were similar for both the left and right side of the face at baseline. By Day 168, the acne scar scale score for the TE implant treated side of the face was significantly higher than that for the Saline Control side (difference=5.438 points, p=0.005). The change from baseline in the acne scar scale was also calculated. Both the TE implant site and Saline Control site showed significant improvement from baseline values (p<0.001 for both sides).

### Example 2: The Use of the TE implant for Acne Scars

For the treatment of acne scars, a composition comprising rH tropoelastin cross-linked with derivatized hyaluronic acid to form a biomaterial matrix (30mg/ml TE cross-linked with 0.5% dHA) was used. The composition was shown to support the repair of atrophic scars during the remodeling and maturation phase to reduce the appearance of the scar.

Patients were treated in areas at a size of 2cm x 2cm which contained at least 2 rolling atrophic scars. The composition was administered at days 0, 28 and 56 for a total volume of 0.5 mls per treatment. A follow up was then performed at days 7, 84 and at day 168. The study was performed on males and females between the ages of 18-55. Efficacy assessments were performed with an Antera^{®} 3D camera.

The patients also served as their own control, using a saline control on another area of skin not receiving the TE composition.

Prior to treatment, the patients reviewed their scars and performed a self-assessment on their acne scars. After treatment, the patients further assessed the appearance of their acne scars. Scar assessment scores were obtained at day 0 in order to perform a comparison at the end of the treatment.

A Miravex Antera 3D camera system was used to enable the analysis of the depression, elevation and color across the treatment field and on a per lesion basis. Treatment fields assessed in a pre-screen visit was performed to ensure bilaterally equivalent treatment fields could be selected.

As shown in the studies, the composition led to surprising results of decreased depth and size of the acne scar areas as well as improved coloring of the skin. For example, patients noticed smoother, less indented, improved texture, as well as improved scar edges.

Surprising results as measured by the 3D camera included a 24% reduction in area of an acne scar by day 168 and 21% by day 336, as compared to control. The TE implant also achieved a 45-50% reduction in contour depression volume and improvement in skin color through day 336 after treatment, as compared to control.

Regarding skin coloring, there was a significant *reduction* in the L* black to white scale in the TE implant treatment field compared to the Control treatment field at both Day 168 and Day 336 and a significant *increase* in the a* green to red color scale in the TE implant treatment field compared to the Control treatment field at both Day 168 and Day 336.

### Further Considerations

In some embodiments, any of the clauses herein may depend from any one of the independent clauses or any one of the dependent clauses. In one aspect, any of the clauses (e.g., dependent or independent clauses) may be combined with any other one or more clauses (e.g., dependent or independent clauses). In one aspect, a claim may include some or all of the words (e.g., steps, operations, means or components) recited in a clause, a sentence, a phrase or a paragraph. In one aspect, a claim may include some or all of the words recited in one or more clauses, sentences, phrases or paragraphs. In one aspect, some of the words in each of the clauses, sentences, phrases or paragraphs may be removed. In one aspect, additional words or elements may be added to a clause, a sentence, a phrase or a paragraph. In one aspect, the subject technology may be implemented without utilizing some of the components, elements, functions or operations described herein. In one aspect, the subject technology may be implemented utilizing additional components, elements, functions or operations.

The subject technology is illustrated, for example, according to various aspects described below. Various examples of aspects of the subject technology are described as numbered clauses (1, 2, 3, etc.) for convenience. These are provided as examples and do not limit the subject technology. It is noted that any of the dependent clauses may be combined in any combination, and placed into a respective independent clause, e.g., clause 1 or clause 20. The other clauses can be presented in a similar manner.

Clause 1. A method of treating an area of skin having an acne scar to improve color and/or appearance, and/or reduce depth, affected area, and/or volume of the acne scar in the area of skin of a patient in need thereof, the method comprising administering a composition that comprises tropoelastin to the area of skin having the acne scar.

Clause 2. The method of Clause 1, wherein the composition comprises between about 1 mg/ml to about 400 mg/ml tropoelastin.

Clause 3. The method of Clause 1 or 2, wherein the composition comprises 1 mg/ml, 5 mg/ml, 10 mg/ml, 20 mg/ml, 30 mg/ml, 40 mg/ml, 50 mg/ml, 60 mg/ml, 70 mg/ml, 80 mg/ml, 90 mg/ml, 100 mg/ml, 110 mg/ml, 120 mg/ml, 130 mg/ml, 140 mg/ml, 150 mg/ml, 160 mg/ml, 170 mg/ml, 180 mg/ml, 190 mg/ml, 200 mg/ml, 210 mg/ml, 220 mg/ml, 230 mg/ml, 240 mg/ml, 250 mg/ml, 260 mg/ml, 270 mg/ml, 280 mg/ml, 290 mg/ml, 300 mg/ml, 310 mg/ml, 320 mg/ml, 330 mg/ml, 340 mg/ml, 350 mg/ml, 360 mg/ml, 370 mg/ml, 380 mg/ml, 390 mg/ml or 400 mg/ml tropoelastin or any amount of tropoelastin in between a range defined by any two aforementioned values.

Clause 4. The method of any one of Clauses 1-3, wherein the composition comprises between about 1 mg/ml to about 300 mg/ml tropoelastin.

Clause 5. The method of any one of Clauses 1-4, wherein the composition comprises between about 1 mg/ml to about 250 mg/ml tropoelastin.

Clause 6. The method of any one of Clauses 1-5, wherein the composition comprises between about 1 mg/ml to about 200 mg/ml tropoelastin.

Clause 7. The method of any one of Clauses 1-6, wherein the composition comprises between about 1 mg/ml to about 150 mg/ml tropoelastin.

Clause 8. The method of any one of Clauses 1-7, wherein the composition comprises between about 1 mg/ml to about 100 mg/ml tropoelastin.

Clause 9. The method of any one of Clauses 1-8, wherein the tropoelastin is crosslinked with about 0.1% to about 10% derivatized hyaluronic acid.

Clause 10. The method of any one of Clauses 1-9, wherein the composition comprises between about 1 to about 100 mg/ml tropoelastin crosslinked with about 0.4% to about 1% derivatized hyaluronic acid (HA).

Clause 11. The method of any one of Clauses 1-9, wherein the composition comprises 30 mg/ml recombinant human tropoelastin crosslinked with 0.5% derivatized hyaluronic acid.

Clause 12. The method of any one of Clauses 1-11, wherein the composition further comprises phosphate buffered saline.

Clause 13. The method of any one of Clauses 1-12, wherein the composition comprising tropoelastin is administered around the scar, such as beneath the scar and/or around edges of the scar.

Clause 14. The method of any one Clauses 1-13, wherein the acne scar is subclassified as an ice pick scar, a boxcar scar or a rolling atrophic scar.

Clause 15. The method of any one of Clauses 1-14, wherein the acne scar is an ice pick scar.

Clause 16. The method of any one of Clauses 1-14, wherein the acne scar is a box scar.

Clause 17. The method of any one of Clauses 1-14, wherein the scar is a rolling atrophic acne scar.

Clause 18. The method of any one of Clauses 1-14, wherein the scar is a hypertrophic scar.

Clause 19. The method of any one of Clauses 1-18, wherein the scar comprises fibrotic tissue around edges or underneath of the scar.

Clause 20. The method of any one of Clauses 1-19, wherein the acne scar has a depth of about 0.1 mm to about 5 mm.

Clause 21. The method of any one of Clauses 1-20, wherein the acne scar has an affected area of about 0.05 mm2 to about 400 mm2.

Clause 22. The method of any one of Clauses 1-21, wherein the acne scar has a volume of about 0.01 mm3 to 2,000 mm3.

Clause 23. The method of any one of Clauses 1-22, wherein the method further comprises a step of disrupting fibrotic strands underneath the acne scar.

Clause 24. The method of Clause 23, wherein the disrupting step is performed prior to administration of the composition that comprises tropoelastin to the patient in need thereof.

Clause 25. The method of Clause 23 or 24, wherein the step of disrupting the fibrotic strands creates a dermal pocket underneath the acne scar.

Clause 26. The method of Clause 25, wherein the step of disrupting is performed with a 18G, 21G, 23G, 25G, 27G, 29G or 30G needle.

Clause 27. The method of Clause 25 or 26, wherein the administering comprises injecting the composition into the dermal pocket.

Clause 28. The method of any one of Clauses 1-27, wherein the composition is administered as an injection underneath the acne scar.

Clause 29. The method of any one of Clauses 1-13, wherein the composition is administered in a volume of about 10 µL to about 100 µL per implant/injection.

Clause 30. The method of any one of Clauses 28-29, wherein the injection is given using a retrograde linear threading technique in a cross-hatching arrangement to disrupt fibrous strands within the acne scar.

Clause 31. The method of Clause 30, wherein the needle is inserted into the area of skin having the acne scar at an angle of about 30° parallel to the skin, and wherein the needle comprises a bevel and the bevel is facing upwards.

Clause 32. The method of Clause 31, wherein the needle is inserted into the area of skin having the acne scar more than one time to break up the fibrous strands and create the dermal pocket.

Clause 33. The method of Clause 32, wherein even pressure is applied to inject the composition at a same time as the needle is withdrawn from the dermal pocket.

Clause 34. The method of any one of Clauses 1-33, wherein the administering of the composition is repeated, and wherein one or more bolus injections into the scar is administered.

Clause 35. The method of any one of Clauses 1-34, wherein a maximum volume of composition administered is between about 100 µL to about 5 mL.

Clause 36. The method of anyone of Clauses 1-35, wherein the maximum volume of composition administered is between about 100 µL to about 500 µL per square cm.

Clause 37. The method of any one of Clauses 1-35, wherein the patient in need thereof has a skin type on a Fitzpatrick skin type scale of I, II, III, IV, V or VI.

Clause 38. The method of any one of Clauses 1-37, wherein the scar comprises a grade of 1, 2, 3, 4 or 5.

Clause 39. The method of any one of Clauses 1-38 wherein the skin comprises a skin color defined by CIE L*a*b* color coordinates, wherein the method further decreases L*.

Clause 40. The method of Clause 39, wherein the method decreases L* by about 1%, about 5%, about 10%, about 15%, about 20%, about 25% or greater than about 50%, or any amount defined by a range in between any two aforementioned values.

Clause 41. The method of Clause 39 or 40, wherein the method further increases a*.

Clause 42. The method of Clause 41, wherein the method increases a* by about 1%, about 5%, about 10%, about 15%, about 20%, about 25% or greater than about 50% or any amount defined by a range in between any two aforementioned values.

Clause 43. The method of any one of Clauses 1-42, wherein the scar comprises a silver coloring prior to administering the composition.

Clause 44. The method of Clause 43, wherein administering the composition decreases the silver coloring and increases red coloring or pink coloring of the scar.

Clause 45. The method of Clause 44, wherein the method decreases L* by about 1%, about 5%, about 10%, about 15%, about 20%, about 25% or greater than about 50% or any amount defined by a range in between any two aforementioned values and increases a* by about 1%, about 5%, about 10%, about 15%, about 20%, about 25% or greater than about 50% or any amount defined by a range in between any two aforementioned values.

Clause 46. The method of any one of Clauses 1-45, wherein the scar comprises a color, wherein the color is different from a natural skin coloring of the patient, and wherein administering the composition results in the color of the scar decreasing or fading in color intensity such that the color of the scar integrates into the natural skin coloring of the patient.

Clause 47. The method of any one of Clauses 1-46, wherein the acne scar is a facial, back, or a torso scar.

Clause 48. The method of any one of Clauses 1-47, wherein the depth, affected area and/or volume of the acne scar is reduced following administering of the composition.

Clause 49. The method of any one of Clauses 1-48, wherein the treatment supports repair of atrophic scarring during skin remodeling and maturation phase to reduce appearance of the acne scar.

Clause 50. The method of any one of Clauses 1-49, wherein the treatment reduces the acne scar area by about 10%, about 20%, about 30%, about 40%, about 50%, about 60%, about 70%, about 80% or greater than about 90% or any amount in between a range described by any two aforementioned values.

Clause 51. The method of any one of Clauses 1-50, wherein the treatment reduces the acne scar volume by about 10%, about 20%, about 30%, about 40%, about 50%, about 60%, about 70%, about 80% or greater than about 90% or any amount in between a range described by any two aforementioned values.

Clause 52. The method of any one of Clauses 1-51, wherein the treatment reduces the acne scar depth by about 10%, about 20%, about 30%, about 40%, about 50%, about 60%, about 70%, about 80% or greater than about 90% or any amount in between a range described by any two aforementioned values.

Clause 53. The method of any one of Clauses 1-52, wherein the treatment reduces depression volume and area of skin contours in the region of skin having the acne scar by about 10%, about 20%, about 30%, about 40%, about 50%, about 60%, about 70%, about 80% or greater than about 90% or any amount in between a range described by any two aforementioned values.

Clause 54. The method of any one of Clauses 1-53, wherein the treatment further improves skin color of the acne scar.

Clause 55. The method of any one of Clauses 1-54, wherein the treatment increases the volume and area of elevations of skin contours in the region of skin having the acne scar by about 10%, about 20%, about 30%, about 40%, about 50%, about 60%, about 70%, about 80% or greater than about 90% or any amount in between a range described by any two aforementioned values.

Clause 56. The method of any one of Clauses 53-55, wherein the treatment levels the skin contours in the area of skin having the acne scar.

Clause 57. The method of any one of Clauses 1-56, wherein the treatment reduces L* black to white scale in the area of skin having the acne scar as compared to a control treatment.

Clause 58. A method of restroring skin countours affected by acne scarring in a subject in need thereof, the method comprising: administering a composition that comprises tropoelastin to an area of skin having skin countours affected by acne scarring.

Clause 59. The method of Clause 58, wherein the composition comprises between about 1 mg/ml to about 400 mg/ml tropoelastin.

Clause 60. The method of Clause 58 or 59, wherein the composition comprises 1 mg/ml, 5 mg/ml, 10 mg/ml, 20 mg/ml, 30 mg/ml, 40 mg/ml, 50 mg/ml, 60 mg/ml, 70 mg/ml, 80 mg/ml, 90 mg/ml, 100 mg/ml, 110 mg/ml, 120 mg/ml, 130 mg/ml, 140 mg/ml, 150 mg/ml, 160 mg/ml, 170 mg/ml, 180 mg/ml, 190 mg/ml, 200 mg/ml, 210 mg/ml, 220 mg/ml, 230 mg/ml, 240 mg/ml, 250 mg/ml, 260 mg/ml, 270 mg/ml, 280 mg/ml, 290 mg/ml, 300 mg/ml, 310 mg/ml, 320 mg/ml, 330 mg/ml, 340 mg/ml, 350 mg/ml, 360 mg/ml, 370 mg/ml, 380 mg/ml, 390 mg/ml or 400 mg/ml tropoelastin or any amount of tropoelastin in between a range defined by any two aforementioned values.

Clause 61. The method of any one of Clauses 58-60, wherein the composition comprises between about 1 mg/ml to about 300 mg/ml tropoelastin.

Clause 62. The method of any one of Clauses 58-61, wherein the composition comprises between about 1 mg/ml to about 250 mg/ml tropoelastin.

Clause 63. The method of any one of Clauses 58-62, wherein the composition comprises between about 1 mg/ml to about 200 mg/ml tropoelastin.

Clause 64. The method of any one of Clauses 58-63, wherein the composition comprises between about 1 mg/ml to about 150 mg/ml tropoelastin.

Clause 65. The method of any one of Clauses 58-64, wherein the composition comprises between about 1 mg/ml to about 100 mg/ml tropoelastin.

Clause 66. The method of any one of Clauses 58-65, wherein the tropoelastin is crosslinked with about 0.1% to about 10% derivatized hyaluronic acid.

Clause 67. The method of any one of Clauses 58-66, wherein the composition comprises between about 1 to about 100 mg/ml tropoelastin crosslinked with about 0.4% to about 1% derivatized hyaluronic acid (HA).

Clause 68. The method of any one of Clauses 58-67, wherein the composition comprises 30 mg/ml recombinant human tropoelastin crosslinked with 0.5% derivatized hyaluronic acid.

Clause 69. The method of any one of Clauses 58-68, wherein the composition is administered around the scar, such as beneath the scar and/or around edges of the scar.

The foregoing description is provided to enable a person skilled in the art to practice the various configurations described herein. While the subject technology has been particularly described with reference to the various figures and configurations, it should be understood that these are for illustration purposes only and should not be taken as limiting the scope of the subject technology.

There may be many other ways to implement the subject technology. Various functions and elements described herein may be partitioned differently from those shown without departing from the scope of the subject technology. Various modifications to these configurations will be readily apparent to those skilled in the art, and generic principles defined herein may be applied to other configurations. Thus, many changes and modifications may be made to the subject technology, by one having ordinary skill in the art, without departing from the scope of the subject technology.

It is understood that the specific order or hierarchy of steps in the processes disclosed is an illustration of exemplary approaches. Based upon design preferences, it is understood that the specific order or hierarchy of steps in the processes may be rearranged. Some of the steps may be performed simultaneously. The accompanying method claims present elements of the various steps in a sample order, and are not meant to be limited to the specific order or hierarchy presented.

As used herein, the phrase "at least one of" preceding a series of items, with the term "and" or "or" to separate any of the items, modifies the list as a whole, rather than each member of the list (i.e., each item). The phrase "at least one of" does not require selection of at least one of each item listed; rather, the phrase allows a meaning that includes at least one of any one of the items, and/or at least one of any combination of the items, and/or at least one of each of the items. By way of example, the phrases "at least one of A, B, and C" or "at least one of A, B, or C" each refer to only A, only B, or only C; any combination of A, B, and C; and/or at least one of each of A, B, and C.

Terms such as "top," "bottom," "front," "rear" and the like as used in this disclosure should be understood as referring to an arbitrary frame of reference, rather than to the ordinary gravitational frame of reference. Thus, a top surface, a bottom surface, a front surface, and a rear surface may extend upwardly, downwardly, diagonally, or horizontally in a gravitational frame of reference.

Furthermore, to the extent that the term "include," "have," or the like is used in the description or the claims, such term is intended to be inclusive in a manner similar to the term "comprise" as "comprise" is interpreted when employed as a transitional word in a claim.

The word "exemplary" is used herein to mean "serving as an example, instance, or illustration." Any embodiment described herein as "exemplary" is not necessarily to be construed as preferred or advantageous over some embodiments.

A reference to an element in the singular is not intended to mean "one and only one" unless specifically stated, but rather "one or more." Pronouns in the masculine (e.g., his) include the feminine and neuter gender (e.g., her and its) and vice versa. The term "some" refers to one or more. Underlined and/or italicized headings and subheadings are used for convenience only, do not limit the subject technology, and are not referred to in connection with the interpretation of the description of the subject technology. Moreover, nothing disclosed herein is intended to be dedicated to the public regardless of whether such disclosure is explicitly recited in the above description.

## Claims

1. A composition comprising tropoelastin crosslinked with about 0.5% derivatized hyaluronic acid for use in treating an area of skin having an acne scar to improve one or more of the following: color, appearance, reduced depth, reduced affected area, and reduced volume of the acne scar, in the area of skin of a patient in need thereof, wherein the composition comprises 30 mg/ml tropoelastin.

2. The composition for use of Claim 1, wherein the composition comprising tropoelastin is administered around the scar, preferably beneath the scar and/or around edges of the scar and/or wherein the acne scar is subclassified as an ice pick scar, a boxcar scar, or a rolling atrophic scar.

3. The composition for use of any one of Claim 1 or 2, wherein the acne scar has a depth of about 0.1 mm to about 5 mm and/or wherein the acne scar has an affected area of about 0.05 mm² to about 400 mm² and/or wherein the acne scar has a volume of about 0.01 mm³ to 2,000 mm³.

4. The composition for use of any one of Claims 1-3, wherein the composition is administered as an injection underneath the acne scar, preferably in a volume of about 10 µL to about 100 µL per implant/injection, more perferably wherein a maximum volume of composition administered is between about 100 µL to about 5 mL.

5. The composition for use of any one of Claims 1-4, wherein the composition is administered as an injection underneath the acne scar wherein a maximum volume of composition administered is about 100 µL to about 500 µL per square cm area of skin.

6. The composition for use of any one of Claims 1-5, wherein the composition is administered on days 0, day 28, and day 56.

7. The composition for use of any one of Claims 1-6, wherein the administering of the composition is repeated, and wherein one or more bolus injections into the scar is administered.

8. The composition for use of any one of Claims 1-7, wherein the patient in need thereof has a skin type on a Fitzpatrick skin type scale of I, II, III, IV, V or VI and/or wherein the scar comprises a grade of 1, 2, 3, 4 or 5.

9. The composition for use of any one of Claims 1-8, wherein the depth, affected area and/or volume of the acne scar is reduced following administering of the composition, preferably wherein the treatment supports repair of atrophic scarring during skin remodeling and maturation phase to reduce appearance of the acne scar.

10. The composition for use of any one of Claims 1-9, wherein the treatment reduces the acne scar area by at least about 10% and/or wherein the treatment reduces the acne scar volume by at least about 10% and/or wherein the treatment reduces the acne scar depth by at least about 10%.

11. The composition for use of any one of Claims 1-10, wherein the treatment reduces depression volume and area of skin contours in the region of skin having the acne scar by at least about 10%, optionally wherein the treatment further improves skin color of the acne scar.

12. The composition for use of any one of Claims 1-11, wherein the treatment increases the volume and area of elevations of skin contours in the region of skin having the acne scar by at least about 10%.

13. The composition for use of Claim 1, wherein the hyaluronic acid is activated and/or modified by adding 1-ethyl-3-(3-dimethylaminopropyl)-carbodiimide (EDC) and/or N-hydroxy-succinimde (NHS).

## Patentansprüche

1. Eine Zubereitung, die mit etwa 0,5 % derivatisierter Hyaluronsäure vernetztes Tropoelastin umfasst, für eine Verwendung bei der Behandlung eines Hautbereichs mit Aknenarben zur Verbesserung von einem/einer oder mehreren von Folgenden: Farbe, Aussehen, verringerte Tiefe, verkleinerter betroffener Bereich und vermindertes Volumen der Aknenarben in dem Hautbereich eines Patienten, der ihrer bedarf, wobei die Zubereitung 30 mg/ml Tropoelastin umfasst.

2. Die Zubereitung für eine Verwendung nach Anspruch 1, wobei die Tropoelastin umfassende Zubereitung um die Narbe herum, vorzugsweise unter die Narbe und/oder um die Ränder der Narbe herum verabreicht wird, und/oder wobei die Aknenarbe als Eispickelnarbe, Boxcar-Narbe oder rollende atrophische Narbe subklassifiziert wird.

3. Die Zubereitung für eine Verwendung nach einem der Ansprüche 1 oder 2, wobei die Aknenarbe eine Tiefe von etwa 0,1 mm bis etwa 5 mm und/oder wobei die Aknenarbe einen betroffenen Bereich von etwa 0,05 mm² bis etwa 400 mm² und/oder wobei die Aknenarbe ein Volumen von etwa 0,01 mm³ bis 2000 mm³ besitzt.

4. Die Zubereitung für eine Verwendung nach einem der Ansprüche 1 bis 3, wobei die Zubereitung in Form einer Injektion unter die Aknenarbe, vorzugsweise mit einem Volumen von etwa 10 µl bis etwa 100 µl pro Implantat/Injektion, verabreicht wird, wobei ein maximales Volumen der verabreichten Zubereitung besonders bevorzugt zwischen etwa 100 µl und etwa 5 ml beträgt.

5. Die Zubereitung für eine Verwendung nach einem der Ansprüche 1 bis 4, wobei die Zubereitung in Form einer Injektion unter die Aknenarbe verabreicht wird, wobei ein maximales Volumen der verabreichten Zubereitung etwa 100 µl bis etwa 500 ml pro Quadratzentimeter Hautbereich beträgt.

6. Die Zubereitung für eine Verwendung nach einem der Ansprüche 1 bis 5, wobei die Zubereitung am Tag 0, Tag 28 und Tag 56 verabreicht wird.

7. Die Zubereitung für eine Verwendung nach einem der Ansprüche 1 bis 6, wobei die Verabreichung der Zubereitung wiederholt wird und wobei eine oder mehrere Bolusinjektionen in die Narbe verabreicht werden.

8. Die Zubereitung für eine Verwendung nach einem der Ansprüche 1 bis 7, wobei der Patient, der ihrer bedarf, einen Hauttyp auf der Fitzpatrick-Hauttypskala von I, II, III, IV, V oder VI hat und/oder wobei die Narbe einen Grad 1, 2, 3, 4 oder 5 umfasst.

9. Die Zubereitung für eine Verwendung nach einem der Ansprüche 1 bis 8, wobei Tiefe, betroffener Bereich und/oder Volumen der Aknenarbe nach der Verabreichung der Zubereitung verringert ist, vorzugsweise wobei die Behandlung die Reparatur einer atrophischen Narbenbildung während der Remodellierungs- und Maturationsphase der Haut unterstützt, um das Erscheinungsbild der Aknenarbe zu reduzieren.

10. Die Zubereitung für eine Verwendung nach einem der Ansprüche 1 bis 9, wobei die Behandlung den Bereich der Aknenarbe um mindestens etwa 10 % und/oder wobei die Behandlung das Volumen der Aknenarbe um mindestens etwa 10 % und/oder wobei die Behandlung die Tiefe der Aknenarbe um mindestens etwa 10 % verringert.

11. Die Zubereitung für eine Verwendung nach einem der Ansprüche 1 bis 10, wobei die Behandlung das Vertiefungsvolumen und den Bereich der Hautkontur in dem Hautbereich mit der Aknenarbe um mindestens etwa 10 % verringert, wahlweise wobei die Behandlung ferner die Hautfarbe der Aknenarbe verbessert.

12. Die Zubereitung für eine Verwendung nach einem der Ansprüche 1 bis 11, wobei die Behandlung das Volumen und den Bereich der Erhöhungen der Hautkontur in dem Hautbereich mit der Aknenarbe um mindestens etwa 10 % vergrößert.

13. Die Zubereitung für eine Verwendung nach Anspruch 1, wobei die Hyaluronsäure durch die Zugabe von 1-Ethyl-3-(3-dimethylaminopropyl)-carbodiimid (EDC) und/oder N-Hydroxysuccinimid (NHS) aktiviert und/oder modifiziert wird.

## Revendications

1. Composition comprenant de la tropoélastine réticulée avec environ 0,5 % d'acide hyaluronique dérivé pour une utilisation dans le traitement d'une zone de peau présentant une cicatrice d'acné afin d'améliorer un ou plusieurs de ce qui suit : couleur, apparence, profondeur réduite, surface affectée réduite et volume réduit de la cicatrice d'acné, dans la zone de la peau d'un patient qui en a besoin, dans laquelle la composition comprend 30 mg/ml de tropoélastine.

2. Composition pour une utilisation selon la revendication 1, dans laquelle la composition comprenant de la tropoélastine est administrée autour de la cicatrice, de préférence sous la cicatrice et/ou autour des bords de la cicatrice et/ou dans laquelle la cicatrice d'acné est sous-classée comme une cicatrice taillée à pic, une cicatrice en cratère, ou une cicatrice atrophique creuse.

3. Composition pour une utilisation selon l'une quelconque des revendications 1 ou 2, dans laquelle la cicatrice d'acné a une profondeur d'environ 0,1 mm à environ 5 mm et/ou dans laquelle la cicatrice d'acné a une surface affectée d'environ 0,05 mm² à environ 400 mm² et/ou dans laquelle la cicatrice d'acné a un volume d'environ 0,01 mm³ à 2 000 mm³.

4. Composition pour une utilisation selon l'une quelconque des revendications 1 à 3, dans laquelle la composition est administrée sous forme d'injection sous la cicatrice d'acné, de préférence dans un volume d'environ 10 µL à environ 100 µL par implant/injection, plus préférablement dans laquelle un volume maximum de composition administrée est compris entre environ 100 µL et environ 5 mL.

5. Composition pour une utilisation selon l'une quelconque des revendications 1 à 4, dans laquelle la composition est administrée sous forme d'injection sous la cicatrice d'acné, dans laquelle le volume maximum de composition administré est d'environ 100 µL à environ 500 µL par cm carré de peau.

6. Composition pour une utilisation selon l'une quelconque des revendications 1 à 5, dans laquelle la composition est administrée au jour 0, au jour 28 et au jour 56.

7. Composition pour une utilisation selon l'une quelconque des revendications 1 à 6, dans laquelle l'administration de la composition est répétée, et dans laquelle une ou plusieurs injections bolus dans la cicatrice sont administrées.

8. Composition pour une utilisation selon l'une quelconque des revendications 1 à 7, dans laquelle le patient qui en a besoin a un type de peau sur l'échelle de Fitzpatrick de I, II, III, IV, V ou VI et/ou dans laquelle la cicatrice a un grade de 1, 2, 3, 4 ou 5.

9. Composition pour une utilisation selon l'une quelconque des revendications 1 à 8, dans laquelle la profondeur, la zone affectée et/ou le volume de la cicatrice d'acné sont réduits après l'administration de la composition, de préférence dans laquelle le traitement favorise la réparation de la cicatrice atrophique pendant la phase de remodelage et de maturation de la peau afin de réduire l'apparence de la cicatrice d'acné.

10. Composition pour une utilisation selon l'une quelconque des revendications 1 à 9, dans laquelle le traitement réduit la surface de la cicatrice d'acné d'au moins 10 % environ et/ou dans laquelle le traitement réduit le volume de la cicatrice d'acné d'au moins 10 % environ et/ou dans laquelle le traitement réduit la profondeur de la cicatrice d'acné d'au moins 10 % environ.

11. Composition pour une utilisation selon l'une quelconque des revendications 1 à 10, dans laquelle le traitement réduit le volume de dépression et la surface de contours de peau dans la région de la peau présentant la cicatrice d'acné d'au moins 10 % environ, éventuellement dans laquelle le traitement améliore en outre la couleur de peau de la cicatrice d'acné.

12. Composition pour une utilisation selon l'une quelconque des revendications 1 à 11, dans laquelle le traitement augmente le volume et la surface des élévations des contours de peau dans la région de peau présentant la cicatrice d'acné d'au moins 10 % environ.

13. Composition pour une utilisation selon la revendication 1, dans laquelle l'acide hyaluronique est activé et/ou modifié par l'ajout de 1-éthyl-3-(3-diméthylaminopropyl)-carbodiimide (EDC) et/ou de N-hydroxy-succinimde (NHS).
